(19) Europäisches Patentamt
European Patent Office
Office européen des brevets

(11) Veröffentlichungsnummer: **0 409 114 B1**

# (12) EUROPÄISCHE PATENTSCHRIFT

(45) Veröffentlichungstag der Patentschrift: **09.11.94**

(21) Anmeldenummer: **90113518.6**

(22) Anmeldetag: **14.07.90**

Die Akte enthält technische Angaben, die nach dem Eingang der Anmeldung eingereicht wurden und die nicht in dieser Patentschrift enthalten sind.

(51) Int. Cl.[5]: **C07D 239/42**, C07D 239/46, C07D 239/47, C07D 251/46, C07C 271/08, C07C 311/65, C07C 331/16, A01N 47/36

## (54) Heterocyclisch substituierte Sulfonylharnstoffe, Verfahren zu ihrer Herstellung und ihre Verwendung als Herbizide oder Pflanzenwachstumsregulatoren.

(30) Priorität: **19.07.89 DE 3923819**
**01.06.90 DE 4017664**

(43) Veröffentlichungstag der Anmeldung:
**23.01.91 Patentblatt 91/04**

(45) Bekanntmachung des Hinweises auf die Patenterteilung:
**09.11.94 Patentblatt 94/45**

(84) Benannte Vertragsstaaten:
**AT BE CH DE DK FR GB GR IT LI NL**

(56) Entgegenhaltungen:
**EP-A- 0 131 258**
**EP-A- 0 353 641**
**DE-A- 3 243 533**
**US-A- 3 267 139**

(73) Patentinhaber: **Hoechst Schering AgrEvo GmbH**
**Gerichtstrasse 27**
**D-13342 Berlin (DE)**

(72) Erfinder: **Löher, Heinz-Josef, Dr.**
**Amselweg 9**
**D-6238 Hofheim am Taunus (DE)**
Erfinder: **Willms, Lothar, Dr.**
**Lindenstrasse 17**
**D-5416 Hillscheid (DE)**
Erfinder: **Frey, Michael, Dr.**
**Meraner Strasse 26a**
**D-8902 Neusäss (DE)**
Erfinder: **Bauer, Klaus, Dr.**
**Doorner Strasse 53d**
**D-6450 Hansau (DE)**
Erfinder: **Bieringer, Hermann, Dr.**
**Eichweg 26**
**D-6239 Eppstein/Taunus (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Es ist bekannt, daß heterocyclisch substituierte Alkylsulfonylharnstoffe herbizide und pflanzenwachstumsregulierende Eigenschaften aufweisen (s. EP-A 061661, EP-A 071958, EP-A 131258, DE-OS 3243533). Diese weisen jedoch zum Teil bei ihrer Anwendung Nachteile auf, wie beispielsweise eine hohe Persistenz oder unzureichende Selektivität in wichtigen Nutzkulturen.

Es wurden nun neue heterocyclische Sulfonylharnstoffe mit vorteilhaften herbiziden Eigenschaften gefunden.

Gegenstand der vorliegenden Erfindung sind Verbindungen der allgemeinen Formel (I)

$$R^1\text{-}Y\text{-}A\text{-}NR^2\text{-}SO_2\text{-}NH\text{-}\overset{\overset{\displaystyle Z}{\|}}{C}\text{-}NR^3R^4 \qquad \text{(I)}$$

worin

A
: eine direkte Bindung oder einen gesättigten oder ungesättigten, unverzweigten oder verzweigten $C_1$-$C_{10}$-Kohlenwasserstoffrest, vorzugsweise eine direkte Bindung oder einen $C_2$-$C_{10}$-Kohlenwasserstoffrest, wie insbesondere einen Rest der Formel $CH_2CH_2$, CRR′, $CH_2CHR$ oder $CH_2CRR'$, wobei R und R′ unabhängig voneinander $C_1$-$C_4$-Alkyl oder $C_2$-$C_4$-Alkenyl bedeuten,

$R^1$
: $C_1$-$C_8$-Alkyl, $C_2$-$C_8$-Alkenyl, $C_2$-$C_8$-Alkinyl, $C_3$-$C_8$-Cycloalkyl, $C_5$-$C_8$-Cycloalkenyl oder einen der vorstehenden fünf Reste, der ein- oder mehrfach durch Halogen oder durch solche Reste substituiert ist, die aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, $C_3$-$C_6$-Cycloalkyl, einem Rest eines drei- bis sechsgliedrigen gesättigten Heterozyklus mit einem Sauerstoffatom im Ring, Furyl, Phenyl und einem Phenylrest, der ein- oder mehrfach durch Reste aus der Gruppe aus Halogen, CN, $C_1$-$C_4$-Alkyl, $C_1$-$C_4$-Alkoxy, $C_1$-$C_4$-Halogenalkyl wie $CF_3$, ($C_1$-$C_4$-Alkoxy)-carbonyl und Nitro substituiert ist, ausgewählt sind, oder Phenyl oder einen Phenylrest, der ein- oder mehrfach durch Reste aus der Gruppe bestehend aus Halogen, CN, $C_1$-$C_4$-Alkyl, $C_1$-$C_4$-Alkoxy, $C_1$-$C_4$-Halogenalkyl wie $CF_3$, ($C_1$-$C_4$-Alkoxy)-carbonyl und Nitro substituiert ist, oder einen Rest der Formel
$-NR^5R^6$, wobei $R^5$ und $R^6$ unabhängig voneinander Wasserstoff oder $C_1$-$C_8$-Alkyl oder einer der Reste $R^5$ bzw. $R^6$ $C_1$-$C_4$-Alkoxy bedeuten oder $R^5$ und $R^6$ gemeinsam eine Alkylenkette $-(CH_2)_n-$ mit $n = 2$ bis 7 bilden;

Y
: S, SO oder $SO_2$, vorzugsweise $SO_2$,

$R^2$
: $C_1$-$C_8$-Alkyloxy, $C_2$-$C_8$-Alkenyloxy, $C_2$-$C_8$-Alkinyloxy, oder einen der vorstehenden 3 Reste, der ein- oder mehrfach durch Halogen oder durch Reste aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, ($C_1$-$C_6$-Alkoxy)-carbonyl, Phenoxycarbonyl, Benzyloxycarbonyl und Phenyl substituiert ist,
$C_3$-$C_8$-Cycloalkyloxy, das unsubstituiert ist oder ein-oder mehrfach durch Halogen oder ein- oder zweifach durch $C_1$-$C_4$-Alkoxy oder $C_1$-$C_4$-Alkylthio substituiert ist, $C_5$-$C_8$-Cycloalkenyloxy, Cyclopropylmethyloxy, Epoxypropyloxy, Furfuryloxy, Tetrahydrofurfuryloxy, Phenoxy-$C_1$-$C_6$-alkyloxy, Phenoxy oder einen der letzten zwei vorstehenden Reste, der im Phenylring durch Halogen, $C_1$-$C_4$-Alkyl, $C_1$-$C_4$-Alkoxy oder Nitro substituiert ist,

$R^3$
: H, $C_1$-$C_8$-Alkyl, $C_2$-$C_8$-Alkenyl, $C_2$-$C_8$-Alkinyl oder $C_1$-$C_4$-Alkoxy,

$R^4$
: einen Rest der Formel

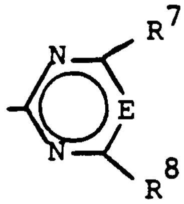 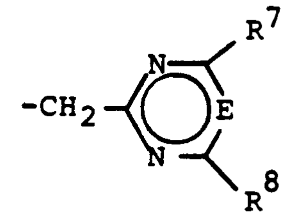 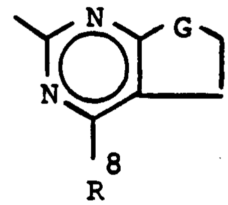

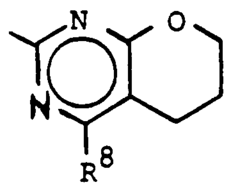 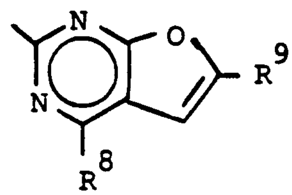 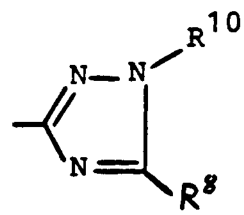

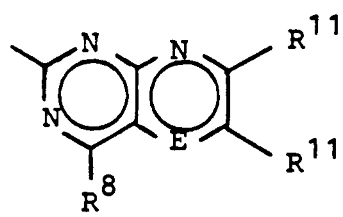 oder 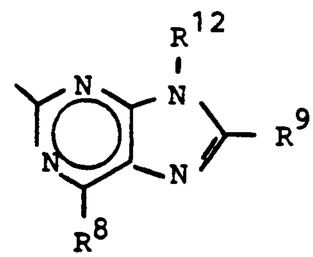

| | |
|---|---|
| $R^7$ und $R^8$ | unabhängig voneinander H, Halogen, $C_1$-$C_6$-Alkyl, $C_1$-$C_6$-Alkoxy, $C_1$-$C_6$-Alkylthio oder einen der letzten drei vorstehenden Reste, der ein- oder mehrfach durch Halogen oder ein- oder zweifach durch $C_1$-$C_4$-Alkoxy oder $C_1$-$C_4$-Alkylthio substituiert ist, oder einen Rest $NR^{13}R^{14}$, $C_3$-$C_6$-Cycloalkyl, $-OCHR^{15}COOR^{16}$, $C_3$-$C_5$-Alkenyl, $C_2$-$C_4$-Alkinyl, $C_3$-$C_5$-Alkenyloxy oder $C_3$-$C_5$-Alkinyloxy, |
| $R^9$ | Wasserstoff oder $C_1$-$C_4$-Alkyl, |
| $R^{10}$ | $C_1$-$C_4$-Alkyl, $-CHF_2$ oder $-CH_2CF_3$, |
| $R^{11}$ | unabhängig voneinander H, $C_1$-$C_4$-Alkyl, $C_1$-$C_4$-Alkoxy oder Halogen, |
| $R^{12}$ | Wasserstoff, $C_1$-$C_4$-Alkyl, $CHF_2$ oder $CH_2CF_3$, |
| $R^{13}$ und $R^{14}$ | unabhängig voneinander H, $C_1$-$C_4$-Alkyl, $C_2$-$C_4$-Alkenyl oder $C_3$-$C_4$-Alkinyl, |
| $R^{15}$ | Wasserstoff oder $C_1$-$C_4$-Alkyl, |
| $R^{16}$ | Wasserstoff oder $C_1$-$C_4$-Alkyl, |
| E | CH oder N, |
| G | $CH_2$ oder O und |
| Z | O oder S, vorzugsweise ein Sauerstoffatom, |

bedeuten, sowie ihre Salze.

Die Verbindungen der Formel (I) können Salze bilden, bei denen der Wasserstoff der $-SO_2$-NH-Gruppe durch ein für die Landwirtschaft geeignetes Kation ersetzt wird. Diese Salze sind beispielsweise Metallsalze, insbesondere Alkali- und Erdalkalimetallsalze, sowie gegebenenfalls alkylierte Ammonium- oder organische Aminsalze. Sie werden vorzugsweise in unter den Reaktionsbedingungen inerten Lösungsmitteln, wie Wasser, Methanol oder Aceton, bei Temperaturen von 0 bis 100 °C aus den Verbindungen der Formel (I) hergestellt. Geeignete Basen zur Herstellung der erfindungsgemäßen Salze sind beispielsweise Alkalicarbonate, wie Kaliumcarbonat, sowie Alkali- und Erdalkalihydroxide, sowie Ammoniak und Ethanolamin.

Von besonderem Interesse sind erfindungsgemäße Verbindungen der Formel (I), in der

$R^1$ $C_1$-$C_4$-Alkyl, einen $C_1$-$C_4$-Alkylrest, der ein- oder mehrfach durch Halogen oder ein- oder zweifach durch $C_1$-$C_4$-Alkoxy, $C_2$-$C_3$-Alkenyloxy, $C_2$-$C_3$-Alkinyloxy, $C_1$-$C_4$-Alkylthio, $C_1$-$C_4$-Alkylsulfinyl, $C_1$-$C_4$-Alkylsulfonyl, Phenyl oder einen Phenylrest, der ein-bis dreifach durch Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, substituiert ist, oder $C_3$-$C_8$-Cycloalkyl oder einen $C_3$-$C_8$-Cycloalkylrest, der ein- oder mehrfach durch Halogen oder ein- bis zweifach durch $C_1$-$C_4$-Alkoxy oder $C_1$-$C_4$-Alkylthio substituiert ist,

oder
$C_5$-$C_8$-Cycloalkenyl, Cyclopropylmethyl, Epoxypropyl, Furfuryl, Tetrahydrofurfuryl, Benzyl, Phenyl, oder einen Benzyl- oder Phenylrest, der im Phenylring durch einen oder mehrere Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist,
einen Rest der Formel $NR^5R^6$, worin $R^5$ $C_1$-$C_4$-Alkyl und $R^6$ Wasserstoff oder $C_1$-$C_4$-Alkyl bedeuten oder $R^5$ und $R^6$ gemeinsam eine Alkylenkette $-(CH_2)_n-$ mit n = 4 oder 5 bilden, bedeutet.

Von besonderem Interesse sind erfindungsgemäße Verbindungen der Formel (I), in der
$R^2$ $C_1$-$C_4$-Alkyloxy, einen $C_1$-$C_4$-Alkyloxyrest, der ein- oder mehrfach durch Halogen oder ein- oder zweifach durch $C_1$-$C_4$-Alkoxy, $C_2$-$C_4$-Alkenyloxy, Propargyloxy, $C_1$-$C_4$-Alkylthio, $C_1$-$C_4$-Alkylsulfinyl, $C_1$-$C_4$-Alkylsulfonyl, ($C_1$-$C_4$-Alkoxy)-carbonyl, Phenoxycarbonyl, Benzyloxycarbonyl oder Phenyl substituiert ist, oder $C_3$-$C_8$-Cycloalkyloxy bedeutet.

Von besonderem Interesse sind weiterhin erfindungsgemäße Verbindungen der Formel (I), in der
$R^4$ einen Rest der Formel

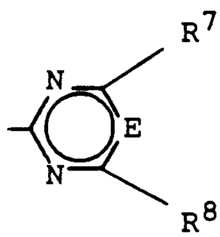

und
$R^7$ und $R^8$ unabhängig voneinander Halogen, $C_1$-$C_4$-Alkyl, $C_1$-$C_4$-Alkoxy, $C_1$-$C_4$-Alkylthio oder einen der letzten drei vorstehenden Reste, die ein- oder mehrfach durch Halogen oder ein- oder zweifach durch $C_1$-$C_4$-Alkoxy, $C_1$-$C_4$-Alkylthio substituiert sind, oder einen Rest $NR^{13}R^{14}$, $C_3$-$C_6$-Cycloalkyl, $-OCHR^{15}COOR^{16}$, Allyl, Propargyl, Allyloxy oder Propargyloxy bedeuten,
$R^{13}$ und $R^{14}$ unabhängig voneinander H oder $C_1$-$C_4$-Alkyl,
$R^{15}$ H oder $C_1$-$C_4$-Alkyl,
$R^{16}$ $C_1$-$C_4$-Alkyl und
E CH oder N bedeuten.

Bevorzugte Verbindungen der Formel (I) sind solche, bei denen
A eine direkte Bindung oder einen Rest der Formel $-CH_2-CH_2-$,
$R^1$ $C_1$-$C_4$-Alkyl, einen $C_1$-$C_4$-Alkylrest, der ein- oder mehrfach durch Halogen oder ein- bis zweifach durch $C_1$-$C_4$-Alkoxy substituiert ist, oder, für den Fall daß A eine direkte Bindung ist, $R^1$ auch vorzugsweise einen Rest der Formel $NR^5R^6$, worin $R^5$ $C_1$-$C_4$-Alkyl und $R^6$ Wasserstoff oder $C_1$-$C_4$-Alkyl bedeuten oder $R^5$ und $R^6$ gemeinsam eine Alkylenkette $-(CH_2)_n-$ mit n = 4 oder 5 bilden, oder, für den Fall daß A = $-CH_2CH_2-$ ist, $R^1$ auch $C_3$-$C_8$-Cycloalkyl, das ein- oder mehrfach durch Halogen substituiert ist oder unsubstituiert ist, Benzyl, Phenyl oder einen Benzyl- oder Phenylrest, der im Phenylring ein- oder mehrfach durch Halogen, $C_1$-$C_4$-Alkyl, $C_1$-$C_4$-Alkoxy, $CF_3$, ($C_1$-$C_4$-Alkoxy)-carbonyl oder Nitro substituiert ist,
$R^2$ $C_1$-$C_4$-Alkoxy, das unsubstituiert ist oder ein- oder mehrfach durch Halogen oder durch ($C_1$-$C_4$-Alkoxy)carbonyl, Phenyloxycarbonyl, Benzyloxycarbonyl oder Phenyl substituiert ist,
$R^3$ H, $C_1$-$C_4$-Alkyl oder Allyl, insbesondere H,
$R^4$ einen Rest der Formel

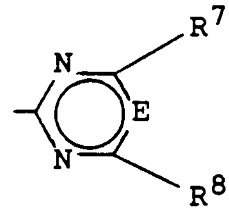

| | |
|---|---|
| $R^7$ und $R^8$ | unabhängig voneinander Halogen, $C_1$-$C_4$-Alkyl, $C_1$-$C_4$-Alkoxy oder einen der letzten beiden vorstehenden Reste, der halogeniert ist, insbesondere die Reste $CH_3$, $OCH_3$, $OC_2H_5$, Cl, $OCF_2H$, $CF_3$, |
| E | CH oder N und |
| Z | O oder S, vorzugsweise ein Sauerstoffatom, bedeuten. |

Ein weiterer Gegenstand der vorliegenden Erfindung ist das Verfahren zur Herstellung von Verbindungen der allgemeinen Formel (I) oder deren Salzen, dadurch gekennzeichnet, daß man

(a) eine Verbindung der Formel (II)

$R^1$-Y-A-$NR^2$-$SO_2$-N = C = Z (II),

mit einer Verbindung der Formel (III)

H-$NR^3R^4$ (III),

umsetzt, wobei in den Formeln (II) und (III) A, Y, Z, $R^1$, $R^2$, $R^3$ und $R^4$ die bei Formel (I) angegebenen Bedeutungen haben, oder

(b) eine Verbindung der Formel (IV)

$R^1$-Y-A-$NR^2$-$SO_2$-$NH_2$ (IV)

mit einem Carbamat bzw. Thiocarbamat der Formel (V)

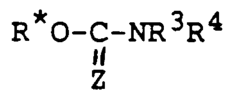 (V)

umsetzt,

wobei in den Formeln (IV) und (V) $R^1$, $R^2$, $R^3$, $R^4$, A, Y und Z die bei Formel (I) angegebenen Bedeutungen haben und $R^*$ $C_1$-$C_6$-Alkyl, $C_1$-$C_4$-Halogenalkyl, Phenyl oder einen Phenylrest bedeutet, der ein- oder mehrfach durch Halogen, $C_1$-$C_4$-Alkyl oder Nitro substituiert ist, oder

(c) ein Carbamat bzw. Thiocarbamat der Formel (VI)

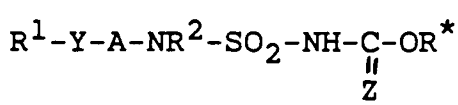 (VI)

mit einer Verbindung der unter (a) genannten Formel (III) umsetzt, wobei $R^1$, $R^2$, $R^*$, Y, A und Z die genannten Bedeutungen haben, oder

(d) eine Verbindung der Formel (VII) oder (VIII)

$R^1$-Y-A-NH-$R^2$ (VII)

$R^1$-Y-A-NH-$R^2$ x HCl (VIII)

mit einer Verbindung der Formel (IX)

$$ClSO_2\text{-}NH\text{-}\underset{\underset{Z}{\|}}{C}\text{-}NR^3R^4 \qquad (IX)$$

umsetzt, wobei in den Formeln (VII) bis (IX) A, Y, Z, $R^1$, $R^2$, $R^3$ und $R^4$ die oben angegebenen Bedeutung haben.

Die Umsetzung der Verbindungen der Formeln (II) und (III) erfolgt vorzugsweise in unter den Reaktionsbedingungen inerten aprotischen Lösungsmitteln, wie beispielsweise Acetonitril, Dichlormethan, Toluol, Tetrahydrofuran oder Dioxan bei Temperaturen von 0 °C bis Siedetemperatur des Reaktionsgemischs. Die Alkylsulfonylisocyanate bzw. -isothiocyanate der Formel (II) sind neu und daher ebenfalls Gegenstand der Erfindung. Sie lassen sich analog üblichen Verfahrensweisen aus den entsprechenden Sulfonamiden der obengenannten Formel (IV) in einfacher Weise herstellen (vgl. z.B. EP-A 085 276). Die Verbindungen der Formel (II) können auch aus den Verbindungen der Formel (VI) durch Reaktion mit Chlorsulfonylisocyanat hergestellt werden (vgl. z.B. DE-A 2 257 240).

Die Ausgangsstoffe der Formel (III) sind bekannt oder lassen sich nach im Prinzip bekannten Verfahrensweisen herstellen, z.B. durch Cyclisierung entsprechender Guanidinderivate mit entsprechend substituierten 1,3-Diketonen; vgl. z.B. "The Chemistry of heterocyclic compounds" Vol. XVI (1962) and Supplement I (1970). Eine andere Möglichkeit besteht in der Derivatisierung von Cyanurchlorid; vgl. z.B. "The Chemistry of Heterocyclic Compounds" L. Rapaport: "s-Triazines and Derivatives" (1959).

Die Umsetzung einer Verbindung (IV) mit einem heterocyclischen Carbamat der Formel (V) wird vorzugsweise in Gegenwart von tertiären organischen Basen, beispielsweise 1,8-Diazabicyclo-[5,4,0]-undec-7-en (DBU), in inerten Lösungsmitteln wie Acetonitril oder Dioxan bei einer Temperatur von 20 °C bis zur Siedetemperatur des Reaktionsgemischs durchgeführt; das Verfahren ist analog dem entsprechenden Verfahren aus EP-A 44807. Die hierzu erforderlichen Carbamate (V) sind literaturbekannt oder werden analog bekannten oder an sich üblichen Verfahren hergestellt; ein entsprechendes Verfahren ist in EP-A 70804 beschrieben.

Die Carbamate der Formel (VI) sind neu und ebenfalls Gegenstand der Erfindung. Sie lassen sich durch Umsetzung der Verbindungen der Formel (IV) mit entsprechenden Chlorameisensäureestern herstellen (vgl. EP-A 87780). Die Umsetzung der Carbamate bzw. Thiocarbamate der Formel (VI) mit den Aminoheterocyclen der Formel (III) führt man vorzugsweise in inerten Lösungsmitteln, beispielsweise Toluol, Xylol, Chlorbenzol, Dioxan und Acetonitril, bei einer Temperatur von 20 °C bis zur Siedetemperatur der betreffenden Reaktionsmischung durch.

Die Verbindungen der Formeln (VII), (VIII) und (IX) lassen sich analog literaturbekannten oder an sich üblichen Verfahren herstellen (vgl. Chem. Ber. 96, 388 (1963); Z. Naturforsch. 36 b, 1673 (1981); J. Am. Chem. Soc. 87, 4359 (1965); Chem. Ber. 118, 564 (1985), US-A 4,016,266 und J. Heterocycl. Chem. 8, 597 (1971)).

Die Sulfonylharnstoffe der Formel (I), welche in den aliphatischen Resten A, $R^1$, $R^2$ ein oder mehrere asymmetrische Kohlenstoffatome enthalten, liegen in enantiomeren und/oder diastereomeren Formen vor. Im allgemeinen werden die entsprechenden erfindungsgemäßen Verbindungen als Racemate oder als Diastereomerengemische erhalten. Falls gewünscht, können die üblichen Techniken zur Trennung dieser Gemische in die sterisch einheitlichen Bestandteile angewendet werden. Auch durch Verwendung von sterisch einheitlichen Ausgangsmaterialien ist eine Reindarstellung der genannten Verbindungen möglich.

Die Formel (I) umfaßt daher alle oben genannten enantiomeren und diastereomeren Formen der oben definierten Verbindungen.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B.. Galium, Viola, Veronica, Lamium, Stellaria, Amaranthus, Sinapis, Ipomoea, Matricaria, Abutilon und Sida auf der annuellen

Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltstoffen und zur Ernteerleichterung wie z.B. durch Auslösen von Desikkation und Wuchstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), konzentrierte Emulsionen (EW), z.B. Öl-in-Wasser- oder Wasser-in-Öl-Emulsionen, versprühbare Lösungen oder Emulsionen, Dispersionen auf Öl- oder Wasserbasis (SC), Stäubemittel (DP), Beizmittel, Granulate (G) wie Boden- bzw. Streuganulate (FG), wasserdispergierbare Granulate (WDG), ULV-Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticides Formulations", Marcel Dekker N.Y., 2nd Ed. 1972-73; K. Martens, "Spray Drying Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v. Olphen, "Introduction to Clay Colloid Chemistry"; 2nd Ed., J. Wiley & Sons, N.Y.; Marsden, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxyethylierte Alkylphenole, polyoxethylierte Fettalkohole, Alkan- oder Alkylarylsulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel z.B. Cyclohexanon, Xylol oder auch höhersiedenden Aromaten oder Kohlenwasserstoffen unter Zusatz von einem oder mehreren Emulgatoren hergestellt. Als Emulgatoren können beispielsweise verwendet werden:

Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Fettalkohol-Propylenoxid-Ethylenoxid-Kondensationsprodukte, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate wie Boden- bzw. Streugranulate oder wasserdispergierbare Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Teller-, Fließbett-, Extruder- und Sprühgranulate können nach üblichen Verfahren hergestellt werden; siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Informationen zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gewichtsprozent, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 80, vorzugsweise 5 bis 80 Gew.-% betragen. Staubförmige Formulierungen enthalten 1 bis 30, vorzugsweise meistens 5 bis 20 Gew.-% an Wirkstoff, versprühbare Lösungen etwa 0,2 bis 25, vorzugsweise 2 bis 20 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden.

Im allgemeinen liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Folgende Beispiele dienen zur Erläuterung der Erfindung:

**FORMULIERUNGSBEISPIELE**

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inerstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether ($^{(R)}$Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexan als Lösungsmittel und 10 Gew.-Teilen oxethyliertes Nonylphenol als Emulgator.

e) Ein in Wasser dispergierbares Granulat wird erhalten, indem man
75 Gewichtsteile einer Verbindung der Formel (I),
10 Gewichtsteile ligninsulfonsaures Calcium,
5 Gewichtsteile Natriumlaurylsulfat,
3 Gewichtsteile Polyvinylalkohol und
7 Gewichtsteile Kaolin
mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man
25 Gewichtsteile einer Verbindung der Formel (I),
5 Gewichtsteile 2,2′-dinaphthylmethan-6,6′-disulfonsaures Natrium
2 Gewichtsteile oleolymethyltaurinsaures Natrium,
1 Gewichtsteile Polyvinylalkohol,
17 Gewichtsteile Calciumcarbonat und
50 Gewichtsteile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

**CHEMISCHE BEISPIELE**

A) N-[(4,6-Dimethoxypyrimidin-2-yl)-aminocarbonyl]-N′-[2-(ethylsulfonyl)-ethyl]-N′-methoxyamino-sulfonamid (vgl. Bsp. 106, Tabelle 1)

a) 2-Ethylsulfonyl-N-methoxyethylamin

Zu 8,5 g 0-Methylhydroxylaminhydrochlorid in 100 ml Methanol gibt man 8,2 g Natriumacetat und läßt 10 Minuten rühren. Anschließend fügt man 9,6 g Vinylethylsulfon zu und erwärmt 2 Stunden auf 50 °C. Anschließend gießt man auf Eiswasser und extrahiert mit Methylenchlorid. Die organische Phase wird einrotiert. Es werden dabei 11 g (82 % d.Th.) eines Rohproduktes erhalten, das ohne weitere Reinigung weiter eingesetzt werden kann.

b) N-[(4,6-Dimethoxy-pyrimidin-2-yl)-aminocarbonyl]-N′-[2-(ethylsulfonyl)-ethyl]-N′-methoxyaminosulfonamid:

3,82 g (0,027 mol) Chlorsulfonylisocyanat werden in 80 ml $CH_2Cl_2$ gelöst und bei -70 °C mit 4,19 g (0,027 mol) 2-Amino-4,6-dimethoxypyrimidin versetzt. Nach 1 Stunde Rühren bei Raumtemperatur wird bei -70 °C ein Gemisch von 4,5 g (0.027 mol) 2-Ethylsulfonyl-N-methoxyethylamin und 2,74 g (0,027 mol) Triethylamin in 50 ml $CH_2Cl_2$ zugetropft. Man rührt 18 Stunden bei Raumtemperatur, extrahiert mit Wasser, trocknet mit $MgSO_4$ und fällt das Produkt mit n-Heptan aus. So erhält man 8,65 g (75 % d.Th.) Produkt mit einem Schmelzpunkt von 170-172 °C.

B) N-[(4,6-Dimethoxy-pyrimidin-2-yl)aminocarbonyl]-N′-[2-(dimethylaminosulfonyl)-eth-1-yl]-N′-methoxyaminosulfonamid(vgl. Bsp. 273, Tabelle 1)

a) 2-Dimethylaminosulfonyl-N-methoxy-ethylamin:

12,37 g (0,148 Mol) 0-Methylhydroxylaminhydrochlorid werden in 120 ml Methanol gelöst und bei Raumtemperatur 10 Minuten mit 12,1 g (0,148 Mol) Natriumacetat gerührt. Anschließend werden 15,96 g (0,118 Mol) Vinylsulfonsäuredimethylamid (hergestellt analog Synthesis 1983, S. 816) - gelöst in 30 ml Methanol - zugetropft. Nach 15 Stunden Rühren bei Raumtemperatur und 3 Stunden bei 50 °C wird die Suspension in Eiswasser gegeben, mehrfach mit $CH_2Cl_2$ extrahiert und der organische Extrakt über $Na_2SO_2$ getrocknet. Man erhält 15,8 g (69,9 % d.Th) eines gelblichen Öl, das ohne weitere Reinigung eingesetzt werden kann.

b) N-[(4,6-Dimethoxy-pyrimidin-2-yl)aminocarbonyl]-N′-[2-(dimethylaminosulfonyl)-eth-1-yl]-N′-methoxyaminosulfonamid:

3,82 g (0,027 Mol) Chlorsulfonylisocyanat wurden in 50 ml $CH_2Cl_2$ und bei -70 °C mit 4,19 g (0.027 mol) 2-Amino-4,6-dimethoxypyrimidin versetzt. Nach 1 Stunde Rühren bei Raumtemperatur wird bei -70 °C ein Gemisch von 4,92 g (0,027 mol) 2-Dimethylaminosulfonyl-N-methoxyethylamin und 2,74 g (0,027 mol) Triethylamin in 50 ml $CH_2Cl_2$ zugetropft. Man rührt 18 Stunden bei Raumtemperatur nach, extrahiert mit Wasser, trocknet über Natriumsulfat und fällt das Produkt mit n-Heptan aus. Man erhält 8,69 g (72,9 % d.Th) an Produkt vom Schmp. 178-180 °C.

In analoger Weise werden die in der folgenden Tabelle 1 definierten Verbindungen erhalten.

**Tabelle 1**

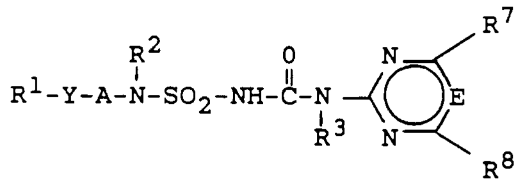

| Bsp Nr. | A | -Y-$R^1$ | $R^2$ | $R^3$ | $R^7$ | $R^8$ | E | Fp. (°C) |
|---|---|---|---|---|---|---|---|---|
| 1 | $-CH_2CH_2-$ | $-S-C_6H_5$ | $-OC_2H_5$ | H | $CH_3$ | $CH_3$ | CH | |
| 2 | " | " | " | H | $OCH_3$ | $CH_3$ | " | |
| 3 | " | " | " | H | $OCH_3$ | $OCH_3$ | " | |
| 4 | " | " | " | H | " | Cl | " | |
| 5 | " | " | " | H | $OCHF_2$ | $CH_3$ | " | |
| 6 | " | " | " | H | " | $CF_3$ | " | |
| 7 | " | " | " | H | " | $OCHF_2$ | " | |
| 8 | " | " | " | H | $CH_3$ | Cl | " | |
| 9 | " | " | " | H | $OCH_3$ | BR | " | |
| 10 | " | " | " | H | " | $NHCH_3$ | " | |
| 11 | " | " | " | H | $OCHF_2$ | $OCH_3$ | " | |
| 12 | " | $-SO_2-C_6H_5$ | " | H | $CH_3$ | $CH_3$ | " | |
| 13 | " | " | " | H | $OCH_3$ | $CH_3$ | " | |
| 14 | " | " | " | H | " | $OCH_3$ | " | |
| 15 | " | " | " | H | " | Cl | " | |
| 16 | " | " | " | H | $OCHF_2$ | $CH_3$ | " | |
| 17 | " | " | " | H | " | $CF_3$ | " | |
| 18 | " | " | " | H | " | $OCHF_2$ | " | |
| 19 | " | " | " | H | $CH_3$ | Cl | " | |
| 20 | " | " | " | H | $OCH_3$ | Br | " | |
| 21 | " | $-SO_2-CH(CH_3)_2$ | $OCH_3$ | H | $OCH_3$ | $NHCH_3$ | " | |
| 22 | " | " | " | H | $OCHF_2$ | $OCH_3$ | " | |
| 23 | " | " | " | H | $CH_3$ | $CH_3$ | " | |
| 24 | " | " | " | H | $OCH_3$ | $CH_3$ | " | 134 |
| 25 | " | " | " | H | " | $OCH_3$ | " | 184-185 |
| 26 | " | " | " | H | " | Cl | " | 193 |
| 27 | " | " | " | H | $OCHF_2$ | $CH_3$ | " | |

Fortsetzung Tabelle 1

| Bsp Nr. | A | $-Y-R^1$ | $R^2$ | $R^3$ | $R^7$ | $R^8$ | E | Fp. (°C) |
|---|---|---|---|---|---|---|---|---|
| 28 | $-CH_2CH_2-$ | $-SO_2-CH(CH_3)_2$ | $-OCH_3$ | H | $OCHF_2$ | $CF_3$ | CH | |
| 29 | " | " | " | H | " | $OCHF_2$ | CH | |
| 30 | " | " | " | H | $CH_3$ | Cl | CH | 62 |
| 31 | " | " | " | H | $OCH_3$ | Br | CH | |
| 32 | " | " | " | H | " | $NHCH_3$ | N | |
| 33 | " | " | " | H | " | $OCH_3$ | N | 159 |
| 34 | " | $-S-C_2H_5$ | " | H | $CH_3$ | $CH_3$ | CH | |
| 35 | " | " | " | H | $OCH_3$ | $CH_3$ | " | |
| 36 | " | " | " | H | " | $OCH_3$ | " | |
| 37 | " | " | " | H | " | Cl | " | |
| 38 | " | " | " | H | $OCHF_2$ | $CH_3$ | " | |
| 39 | " | " | " | H | " | $CF_3$ | " | |
| 40 | " | " | " | H | " | $OCHF_2$ | " | |
| 41 | " | " | " | H | $CH_3$ | Cl | " | |
| 42 | " | " | " | H | $OCH_3$ | Br | " | |
| 43 | " | " | " | H | " | $NHCH_3$ | " | |
| 44 | " | " | " | H | $OCHF_2$ | $OCH_3$ | " | |
| 45 | " | $-SO_2-C_6H_{11}$ | " | H | $OCH_3$ | $OCH_3$ | N | |
| 46 | " | " | " | H | $OCHF_2$ | $OCHF_2$ | N | |
| 47 | " | " | " | H | $CH_3$ | $CH_3$ | N | |
| 48 | " | $-SC_6H_{11}$ | " | H | $OCH_3$ | $OCH_3$ | N | |
| 49 | " | " | " | H | $CH_3$ | $OCHF_2$ | N | |
| 50 | " | $-S-CH_2-C_6H_5$ | " | H | $CH_3$ | $CH_3$ | CH | |
| 51 | " | " | " | H | $OCH_3$ | $CH_3$ | " | |
| 52 | " | " | " | H | $OCH_3$ | $OCH_3$ | " | |
| 53 | " | " | " | H | $OCH_3$ | Cl | " | |
| 54 | " | " | " | H | $OCHF_2$ | $CH_3$ | " | |
| 55 | " | " | " | H | $OCHF_2$ | $CF_3$ | " | |
| 56 | " | " | " | H | $OCHF_2$ | $OCHF_2$ | " | |

Fortsetzung Tabelle 1

| Bsp Nr. | A | -Y-$R^1$ | $R^2$ | $R^3$ | $R^7$ | $R^8$ | E | Fp. (°C) |
|---|---|---|---|---|---|---|---|---|
| 57 | $-CH_2CH_2-$ | $-S-CH_2C_6H_5$ | $-OCH_3$ | H | $CH_3$ | Cl | CH | |
| 58 | " | " | " | H | $OCH_3$ | Br | " | |
| 59 | " | " | " | H | $OCH_3$ | $NHCH_3$ | " | |
| 60 | " | " | " | H | $OCHF_2$ | $OCH_3$ | " | |
| 61 | " | " | $-OCH(CH_3)_2$ | H | $CH_3$ | $CH_3$ | " | |
| 62 | " | " | " | H | $OCH_3$ | $CH_3$ | " | |
| 63 | " | " | " | H | $OCH_3$ | $OCH_3$ | " | |
| 64 | " | " | " | H | $OCH_3$ | Cl | " | |
| 65 | " | " | " | H | $OCHF_2$ | $CH_3$ | " | |
| 66 | " | " | " | H | " | $CF_3$ | " | |
| 67 | " | " | " | H | " | $OCHF_2$ | " | |
| 68 | " | " | " | H | $CH_3$ | Cl | " | |
| 69 | " | " | " | H | $OCH_3$ | Br | " | |
| 70 | " | " | " | H | $OCH_3$ | $NHCH_3$ | " | |
| 71 | " | " | " | H | $OCHF_2$ | $OCH_3$ | " | |
| 72 | " | $-SO_2-CH_3$ | $-OCH_3$ | $CH_3$ | $OCH_3$ | $OCH_3$ | N | |
| 73 | " | " | " | " | $CH_3$ | $OCHF_2$ | " | |
| 74 | " | " | " | H | $OCH_3$ | $OCH_3$ | " | 158 |
| 75 | " | " | " | $CH_3$ | $OCH_3$ | $CF_3$ | " | |
| 76 | " | $-SO_2-CH_2C_6H_5$ | " | H | $CH_3$ | $CH_3$ | " | |
| 77 | " | " | " | " | $OCH_3$ | $CH_3$ | " | |
| 78 | " | " | " | " | $OCH_3$ | $OCH_3$ | " | |
| 79 | " | " | " | " | $OCH_3$ | Cl | " | |
| 80 | " | " | " | " | $OCHF_2$ | $CH_3$ | " | |
| 81 | " | " | " | " | $OCHF_2$ | $CF_3$ | " | |
| 82 | " | " | " | " | $OCHF_2$ | $OCHF_2$ | " | |
| 83 | " | " | " | " | $CH_3$ | Cl | " | |
| 84 | " | " | " | " | $OCH_3$ | Br | " | |
| 85 | " | " | " | " | $OCH_3$ | $NHCH_3$ | " | |

Fortsetzung Tabelle 1

| Bsp Nr. | A | $-Y-R^1$ | $R^2$ | $R^3$ | $R^7$ | $R^8$ | E | Fp. (°C) |
|---|---|---|---|---|---|---|---|---|
| 86 | $-CH_2CH_2-$ | $-SO_2CH_2C_6H_5$ | $-OCH_3$ | H | $OCHF_2$ | $OCH_3$ | N | |
| 87 | " | $-S-CH_3$ | $-OC_2H_5$ | H | $CH_3$ | $CH_3$ | " | |
| 88 | " | " | " | " | $OCH_3$ | $CH_3$ | " | |
| 89 | " | " | " | " | $OCH_3$ | $OCH_3$ | " | |
| 90 | " | " | " | " | $OCH_3$ | Cl | " | |
| 91 | " | " | " | " | $OCHF_2$ | $CH_3$ | " | |
| 92 | " | " | " | " | $OCHF_2$ | $CF_3$ | " | |
| 93 | " | " | " | " | $OCHF_2$ | $OCHF_2$ | " | |
| 94 | " | " | " | " | $CH_3$ | Cl | " | |
| 95 | " | " | " | " | $OCH_3$ | Br | " | |
| 96 | " | " | " | " | $OCH_3$ | $NHCH_3$ | " | |
| 97 | " | " | " | " | $OCHF_2$ | $OCH_3$ | " | |
| 98 | " | $-SO_2C_2H_5$ | $-OCH(CH_3)_2$ | " | $OCHF_2$ | $CF_3$ | CH | |
| 99 | " | " | " | " | $OCHF_2$ | $OCHF_2$ | " | |
| 100 | " | " | " | " | $CH_3$ | Cl | " | |
| 101 | " | " | " | " | $OCH_3$ | Br | " | |
| 102 | " | " | " | " | $OCH_3$ | $NHCH_3$ | " | |
| 103 | " | " | " | " | $OCHF_2$ | $OCH_3$ | " | |
| 104 | " | " | $-OCH_3$ | H | $CH_3$ | $CH_3$ | " | |
| 105 | " | " | " | " | $OCH_3$ | $CH_3$ | " | 138 |
| 106 | " | " | " | " | $OCH_3$ | $OCH_3$ | " | 170-172 |
| 107 | " | " | " | " | $OCH_3$ | Cl | " | 172 |
| 108 | " | " | $-OCH_2CH_2CH_3$ | " | $CH_3$ | $CH_3$ | " | |
| 109 | " | " | " | " | $OCH_3$ | $CH_3$ | " | |
| 110 | " | " | " | " | $OCH_3$ | $OCH_3$ | " | |
| 111 | " | " | " | " | $OCH_3$ | Cl | " | |
| 112 | " | " | " | " | $OCHF_2$ | $CH_3$ | " | |
| 113 | " | " | " | " | $OCHF_2$ | $CF_3$ | " | |
| 114 | " | " | " | " | $OCHF_2$ | $OCHF_2$ | " | |

Fortsetzung Tabelle 1

| Bsp Nr. | A | -Y-$R^1$ | $R^2$ | $R^3$ | $R^7$ | $R^8$ | E | Fp. (°C) |
|---|---|---|---|---|---|---|---|---|
| 115 | $-CH_2CH_2-$ | $-SO_2C_2H_5$ | $-OCH_2CH_2CH_3$ | H | $CH_3$ | Cl | CH | |
| 116 | " | " | " | " | $OCH_3$ | Br | " | |
| 117 | " | " | " | " | $OCH_3$ | $NHCH_3$ | " | |
| 118 | " | " | " | " | $OCHF_2$ | $OCH_3$ | " | |
| 119 | " | " | $-OCH(CH_3)_2$ | " | $CH_3$ | $CH_3$ | " | |
| 120 | " | " | " | " | $OCH_3$ | $CH_3$ | " | |
| 121 | " | " | " | " | $OCH_3$ | $OCH_3$ | " | |
| 122 | " | " | " | " | $OCH_3$ | Cl | " | |
| 123 | " | " | " | " | $OCHF_2$ | $CH_3$ | " | |
| 124 | " | " | $-O(CH_2)_3CH_3$ | " | $OCH_3$ | $NHCH_3$ | " | |
| 125 | " | " | " | " | $OCHF_2$ | $OCH_3$ | " | |
| 126 | " | " | $-OCH_3$ | " | $OCHF_2$ | $CH_3$ | " | |
| 127 | " | " | " | " | " | $CF_3$ | " | |
| 128 | " | " | " | " | " | $OCHF_2$ | " | |
| 129 | " | " | " | " | $CH_3$ | Cl | " | 59-62 |
| 130 | " | " | " | " | $OCH_3$ | Br | " | |
| 131 | " | " | " | " | $OCH_3$ | $NHCH_3$ | " | |
| 132 | " | " | " | " | $OCHF_2$ | $OCH_3$ | " | |
| 133 | " | " | $-O(CH_2)_3CH_3$ | " | $CH_3$ | $CH_3$ | " | |
| 134 | " | " | " | " | $OCH_3$ | $CH_3$ | " | |
| 135 | " | " | " | " | $OCH_3$ | $OCH_3$ | " | |
| 136 | " | " | " | " | $OCH_3$ | Cl | " | |
| 137 | " | " | " | " | $OCHF_2$ | $CH_3$ | " | |
| 138 | " | " | " | " | $OCHF_2$ | $CF_3$ | " | |
| 139 | " | " | " | " | $OCHF_2$ | $OCHF_2$ | " | |
| 140 | " | " | " | " | $CH_3$ | Cl | " | |
| 141 | " | " | " | " | $OCH_3$ | Br | " | |
| 142 | " | " | $-OC_6H_{11}$ | " | $CH_3$ | $CH_3$ | " | |
| 143 | " | " | " | " | $OCH_3$ | $CH_3$ | " | |
| 144 | " | " | " | " | $OCH_3$ | $OCH_3$ | " | |

Fortsetzung Tabelle 1

| Bsp Nr. | A | -Y-$R^1$ | $R^2$ | $R^3$ | $R^7$ | $R^8$ | E | Fp. (°C) |
|---|---|---|---|---|---|---|---|---|
| 145 | $-CH_2CH_2-$ | $-SO_2C_2H_5$ | $-OC_6H_{11}$ | H | $OCH_3$ | Cl | CH | |
| 146 | " | " | " | " | $OCHF_2$ | $CH_3$ | " | |
| 147 | " | " | " | " | $OCHF_2$ | $CF_3$ | " | |
| 148 | " | " | " | " | $OCHF_2$ | $OCHF_2$ | " | |
| 149 | " | " | " | " | $CH_3$ | Cl | " | |
| 150 | " | " | " | " | $OCH_3$ | Br | " | |
| 151 | " | " | " | " | $OCH_3$ | $NHCH_3$ | " | |
| 152 | " | " | " | " | $OCHF_2$ | $OCH_3$ | " | |
| 153 | " | $-SO_2CH_3$ | $-OC_3H_7$ | " | $CH_3$ | $CH_3$ | " | |
| 154 | " | " | " | " | $OCH_3$ | $CH_3$ | " | |
| 155 | " | " | " | " | " | $OCH_3$ | " | |
| 156 | " | " | " | " | " | Cl | " | |
| 157 | " | " | " | " | $OCHF_2$ | $CH_3$ | " | |
| 158 | " | " | " | " | $OCHF_2$ | $CF_3$ | " | |
| 159 | " | " | " | " | $OCHF_2$ | $OCHF_2$ | " | |
| 160 | " | " | " | " | $CH_3$ | Cl | " | |
| 161 | " | " | " | " | $OCH_3$ | Br | " | |
| 162 | " | " | " | " | $OCH_3$ | $NHCH_3$ | " | |
| 163 | " | " | " | " | $OCHF_2$ | $OCH_3$ | " | |
| 164 | " | " | $-OCH_2CH_3$ | " | $CH_3$ | $CH_3$ | " | |
| 165 | " | " | " | " | $OCH_3$ | $CH_3$ | " | 145-147 |
| 166 | " | " | " | " | " | $OCH_3$ | " | 160-163 |
| 167 | " | " | " | " | " | Cl | " | 178 |
| 168 | " | " | " | " | $OCHF_2$ | $CH_3$ | " | |
| 169 | " | " | " | " | " | $CF_3$ | " | |
| 170 | " | " | " | " | " | $OCHF_2$ | " | |
| 171 | " | " | " | " | $CH_3$ | Cl | " | 50-51 |
| 172 | " | " | " | " | $OCH_3$ | Br | " | |
| 173 | " | " | " | " | $OCH_3$ | $NHCH_3$ | " | |
| 174 | " | " | " | " | $OCHF_2$ | $OCH_3$ | " | |

Fortsetzung Tabelle 1

| Bsp Nr. | A | -Y-$R^1$ | $R^2$ | $R^3$ | $R^7$ | $R^8$ | E | Fp. (°C) |
|---|---|---|---|---|---|---|---|---|
| 175 | $-CH_2CH_2-$ | $-SO_2CH_2CH_3$ | $-OC_6H_5$ | H | $CH_3$ | $CH_3$ | CH | |
| 176 | " | " | " | " | $CH_3$ | $OCH_3$ | " | |
| 177 | " | " | " | " | $OCH_3$ | $OCH_3$ | " | |
| 178 | " | " | " | " | $OCH_3$ | $OCH_3$ | N | |
| 179 | " | " | " | " | $CH_3$ | $OCH_3$ | N | |
| 180 | " | " | " | " | $OCHF_2$ | $OCHF_2$ | CH | |
| 181 | " | $-SO_2C_6H_5$ | $-O-CH_3$ | " | $CH_3$ | $CH_3$ | " | |
| 182 | " | " | " | " | $CH_3$ | $OCH_3$ | " | |
| 183 | " | " | " | " | $OCH_3$ | $OCH_3$ | " | |
| 184 | " | " | " | " | $OCH_3$ | $OCH_3$ | N | |
| 185 | " | " | " | " | $CH_3$ | $OCH_3$ | N | |
| 186 | " | " | " | " | $OCHF_2$ | $OCH_3$ | CH | |
| 187 | " | $-SO_2N(CH_3)_2$ | " | " | $CH_3$ | $CH_3$ | " | 145-147 |
| 188 | " | " | " | " | $CH_3$ | $OCH_3$ | " | 162-164 |
| 189 | " | " | " | " | $OCH_3$ | Cl | " | 168-171 |
| 190 | " | " | " | " | $OCH_3$ | $OCH_3$ | N | 124 |
| 191 | " | " | " | " | $CH_3$ | $OCH_3$ | N | 112-113 |
| 192 | " | " | " | " | $OCHF_2$ | $OCHF_2$ | CH | 88-89 |
| 193 | " | " | $-OC_2H_5$ | " | $CH_3$ | $CH_3$ | " | |
| 194 | " | " | " | " | $CH_3$ | $OCH_3$ | " | |
| 195 | " | " | " | " | $OCH_3$ | $OCH_3$ | " | |
| 196 | " | " | " | " | $OCH_3$ | $OCH_3$ | N | |
| 197 | " | " | " | " | $CH_3$ | $OCH_3$ | N | |
| 198 | " | " | " | " | $OCHF_2$ | $OCHF_2$ | CH | |
| 199 | " | " | $-OC_3H_7(n)$ | " | $OCH_3$ | $OCH_3$ | " | |
| 200 | " | " | " | " | $OCH_3$ | $OCH_3$ | N | |
| 201 | " | " | " | " | $CH_3$ | $OCH_3$ | CH | |
| 202 | " | " | " | " | $CH_3$ | $OCH_3$ | N | |
| 203 | " | " | $-OC_4H_9(n)$ | " | $OCH_3$ | $OCH_3$ | CH | |
| 204 | " | " | " | " | $OCH_3$ | Cl | " | |

Fortsetzung Tabelle 1

| Bsp Nr. | A | $-Y-R^1$ | $R^2$ | $R^3$ | $R^7$ | $R^8$ | E | Fp. (°C) |
|---|---|---|---|---|---|---|---|---|
| 205 | $-CH_2CH_2-$ | $-SO_2NHCH_3$ | $OCH_3$ | H | $OCH_3$ | $OCH_3$ | CH | |
| 206 | " | " | " | " | $OCH_3$ | Cl | " | |
| 207 | " | " | " | " | $OCH_3$ | $CH_3$ | " | |
| 208 | " | " | " | " | $CH_3$ | $CH_3$ | " | |
| 209 | " | " | " | " | $CH_3$ | $OCH_3$ | N | |
| 210 | " | " | " | " | $OCH_3$ | $OCH_3$ | N | |
| 211 | " | $-SO_2NHC_2H_5$ | " | " | $OCH_3$ | $OCH_3$ | CH | |
| 212 | " | " | " | " | $OCH_3$ | Cl | " | |
| 213 | " | " | " | " | $OCH_3$ | $CH_3$ | N | |
| 214 | " | " | " | " | $OCH_3$ | $OCH_3$ | N | |
| 215 | " | $-SO_2$ 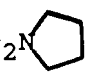 | " | " | $OCH_3$ | $OCH_3$ | CH | |
| 216 | " | " | " | $CH_3$ | $OCH_3$ | $OCH_3$ | " | |
| 217 | " | " | " | H | $OCH_3$ | $CH_3$ | " | |
| 218 | " | $-SO_2$ 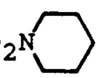 | " | " | $OCH_3$ | $CH_3$ | " | |
| 219 | " | " | " | " | $OCH_3$ | $OCH_3$ | " | |
| 220 | " | $-SO_2C_2H_5$ | " | $CH_3$ | $OCH_3$ | $OCH_3$ | " | |
| 221 | " | " | " | $CH_3$ | $OCH_3$ | $CH_3$ | N | |
| 222 | " | $-SO_2-N(CH_3)_2$ | " | $CH_3$ | $OCH_3$ | $OCH_3$ | CH | |
| 223 | " | " | " | $CH_3$ | $OCH_3$ | $CH_3$ | N | |
| 224 | " | $-SO_2NHCH_3$ | " | $CH_3$ | $OCH_3$ | $OCH_3$ | CH | |
| 225 | " | $-SO_2CH(CH_3)_2$ | $OC_2H_5$ | H | $OCH_3$ | $OCH_3$ | CH | 175 |
| 226 | " | " | " | " | $OCH_3$ | $CH_3$ | CH | 50 |
| 227 | " | " | " | " | Cl | $CH_3$ | CH | 56- 60 |
| 228 | " | " | " | " | $OCH_3$ | $OCH_3$ | N | 162 |

Fortsetzung Tabelle 1

| Bsp Nr. | A | $-Y-R^1$ | $R^2$ | $R^3$ | $R^7$ | $R^8$ | E | Fp. (°C) |
|---|---|---|---|---|---|---|---|---|
| 229 | $-CH_2CH_2-$ | $-SO_2C_2H_5$ | $OCH_3$ | H | Cl | $CH_3$ | CH | 59- 62 |
| 230 | " | " | " | " | $OCH_3$ | $OCH_3$ | N | 176-178 |
| 231 | " | " | $OC_2H_5$ | " | " | " | CH | 180-182 |
| 232 | " | " | " | " | " | $CH_3$ | CH | 51- 53 |
| 233 | " | " | " | " | Cl | $CH_3$ | CH | 55- 60 |
| 234 | " | " | " | " | $OCH_3$ | $OCH_3$ | N | 172-174 |
| 235 | " | " | " | " | $OCHF_2$ | $OCHF_2$ | CH | |
| 236 | " | " | " | " | " | $CH_3$ | CH | |
| 237 | $CH_2CH_2$ | $SO_2CH_3$ | $OCH_3$ | H | $OCH_3$ | $OCH_3$ | CH | 184 |
| 238 | " | " | " | " | " | $CH_3$ | " | 152-153 |
| 239 | " | " | " | " | Cl | " | " | 49- 50 |
| 240 | " | " | $OC_2H_5$ | " | $OCH_3$ | $OCH_3$ | N | 155-156 |
| 241 | $-CH_2CH_2-$ | $-SO_2(n)-C_3H_7$ | $OCH_3$ | H | $CH_3$ | $CH_3$ | CH | 129 |
| 242 | " | " | " | " | $OCH_3$ | $OCH_3$ | CH | 160 |
| 243 | " | " | " | " | $OCH_3$ | $CH_3$ | CH | |
| 244 | " | " | " | " | $OCH_3$ | $OCH_3$ | N | |
| 245 | " | " | $OC_2H_5$ | " | $OCH_3$ | $OCH_3$ | CH | |
| 246 | " | " | " | " | $OCH_3$ | $OCH_3$ | N | |
| 247 | " | " | " | " | $OCH_3$ | $CH_3$ | CH | |
| 248 | " | " | " | " | $CH_3$ | $CH_3$ | CH | |
| 249 | " | $-SO_2C_6H_5$ | $OCH_3$ | " | $OCH_3$ | $OCH_3$ | CH | |
| 250 | " | " | " | " | $OCH_3$ | $OCH_3$ | N | |
| 251 | " | " | " | " | $OCH_3$ | $CH_3$ | CH | |
| 252 | " | " | " | " | $CH_3$ | $CH_3$ | CH | |
| 253 | " | " | $OC_2H_5$ | " | $OCH_3$ | $OCH_3$ | CH | |
| 254 | " | " | " | " | $OCH_3$ | $OCH_3$ | N | |
| 255 | " | " | " | " | $OCH_3$ | $CH_3$ | CH | |
| 256 | " | " | " | " | $CH_3$ | $CH_3$ | CH | |

Fortsetzung Tabelle 1

| Bsp Nr. | A | $-Y-R^1$ | $R^2$ | $R^3$ | $R^7$ | $R^8$ | E | Fp. (°C) |
|---|---|---|---|---|---|---|---|---|
| 257 | $-CH_2CH_2-$ | $-SOC_2H_5$ | $OCH_3$ | H | $OCH_3$ | $OCH_3$ | CH | |
| 258 | " | " | " | " | $OCH_3$ | $OCH_3$ | N | |
| 259 | " | " | " | " | $OCH_3$ | $CH_3$ | CH | |
| 260 | " | " | " | " | $CH_3$ | $CH_3$ | CH | |
| 261 | " | " | $OC_2H_5$ | " | $OCH_3$ | $OCH_3$ | CH | |
| 262 | " | " | " | " | $OCH_3$ | $OCH_3$ | N | |
| 263 | " | " | " | " | $OCH_3$ | $CH_3$ | CH | |
| 264 | " | " | " | " | $CH_3$ | $CH_3$ | CH | |
| 265 | " | $-SOCH_3$ | $OCH_3$ | " | $OCH_3$ | $OCH_3$ | CH | |
| 266 | " | " | " | " | $OCH_3$ | $OCH_3$ | N | |
| 267 | " | " | " | " | $OCH_3$ | $CH_3$ | CH | |
| 268 | " | " | " | " | $CH_3$ | $CH_3$ | CH | |
| 269 | " | " | $OC_2H_5$ | " | $OCH_3$ | $OCH_3$ | CH | |
| 270 | " | " | " | " | $OCH_3$ | $OCH_3$ | N | |
| 271 | " | " | " | " | $OCH_3$ | $CH_3$ | CH | |
| 272 | " | " | " | " | $CH_3$ | $CH_3$ | CH | |
| 273 | " | $-SO_2N(CH_3)_2$ | $OCH_3$ | " | $OCH_3$ | $OCH_3$ | CH | 178-180 |
| 275 | " | $-SO_2N(C_2H_5)_2$ | " | " | $OCH_3$ | $OCH_3$ | CH | |
| 276 | " | " | " | " | $CH_3$ | $CH_3$ | CH | |
| 277 | " | " | " | " | $OCH_3$ | $CH_3$ | CH | |
| 278 | " | " | " | " | $OCH_3$ | $OCH_3$ | N | |
| 279 | " | " | $OC_2H_5$ | " | $OCH_3$ | $OCH_3$ | CH | |
| 280 | " | " | $OC_6H_5$ | " | $OCH_3$ | $OCH_3$ | CH | |
| 281 | " | " | " | " | $OCH_3$ | Cl | CH | |
| 282 | " | $-SO_2N(C_3H_7)_2$ | $OCH_3$ | " | $OCH_3$ | $OCH_3$ | CH | |
| 283 | " | $-SO_2NH(n-C_4H_9)$ | $OCH_3$ | " | $OCH_3$ | $OCH_3$ | CH | |
| 284 | " | $-SO_2-NH-OCH_3$ | $OCH_3$ | " | $OCH_3$ | $OCH_3$ | CH | |
| 285 | " | $-SO_2-NH-OC_2H_5$ | $OCH_3$ | " | $OCH_3$ | $OCH_3$ | CH | |
| 286 | " | $-SO_2N(OCH_3)(CH_3)$ | $OCH_3$ | " | $OCH_3$ | $OCH_3$ | CH | |

Fortsetzung Tabelle 1

| Bsp Nr. | A | $-Y-R^1$ | $R^2$ | $R^3$ | $R^7$ | $R^8$ | E | Fp. (°C) |
|---|---|---|---|---|---|---|---|---|
| 287 | $CH_2CH_2CH_2$ | $-SO_2C_2H_5$ | $OCH_3$ | H | $OCH_3$ | $OCH_3$ | CH | |
| 288 | " | " | $OCH_3$ | " | $CH_3$ | $CH_3$ | CH | |
| 289 | " | " | $OCH_3$ | " | $OCH_3$ | $CH_3$ | CH | |
| 290 | " | " | $OCH_3$ | " | $OCH_3$ | Cl | CH | |
| 291 | " | " | $OCH_3$ | $CH_3$ | $OCH_3$ | $OCH_3$ | CH | |
| 292 | " | " | $OCH_3$ | $CH_3$ | $OCH_3$ | $OCH_3$ | N | |
| 293 | " | $-SO_2N(CH_3)_2$ | $OCH_3$ | H | $OCH_3$ | $OCH_3$ | CH | |
| 294 | " | " | $OCH_3$ | H | $CH_3$ | $CH_3$ | CH | |
| 295 | $-CH_2CH_2CH_2CH_2-$ | $SO_2CH_3$ | " | H | $OCH_3$ | $OCH_3$ | CH | |
| 296 | $-CH_2-$ | " | " | H | $OCH_3$ | $OCH_3$ | CH | |
| 297 | $-CH(CH_3)-$ | " | " | H | $OCH_3$ | $OCH_3$ | CH | |
| 298 | $-CH_2CH(CH_3)-$ | $SO_2C_2H_5$ | " | H | $OCH_3$ | $OCH_3$ | CH | |
| 299 | $-C(CH_3)_2-$ | " | " | H | $CH_3$ | $CH_3$ | CH | |
| 300 | " | " | " | H | $OCH_3$ | $OCH_3$ | CH | |

N-[(4,6-Dimethoxy-pyrimidin-2-yl)-aminocarbonyl]-N'-(methylsulfonyl)-N'-(methoxy)-aminosulfonamid (vgl. Bsp. 473, Tabelle 2)

a) N-Methylsulfonyl-methoxyamino-sulfonylisocyanat:
3,4 g (0,0272 Mol) O-Methyl-N-methylsulfonyl-hydroxylamin (hergestellt nach Z. Naturforsch. 36b, 1673 (1981)) werden in 80 ml wasserfreiem Chlorbenzol suspendiert und bei 0 °C mit 2,53 ml (4,10 g; 0,029 Mol) Chlorsulfonylisocyanat versetzt. Anschließend wird das Reaktionsgemisch langsam unter Durchleiten von Stickstoff aufgeheizt, wobei die Suspension bei 40 °C in eine klare Lösung übergeht. Nach ca. 3stündigem Erhitzen unter Rückfluß wird das Gemisch abgekühlt, einrotiert und im Hochvakuum getrocknet. Man erhält 6,18 g (99 % d.Th.) an N-Methylsulfonyl-methoxyamino-sulfonylisocyanat, das ohne weitere Reinigung in der nächsten Stufe eingesetzt werden kann.
b) N-[(4,6-Dimethoxy-pyrimidin-2-yl)aminocarbonyl]-N'-(methylsulfonyl)-N'-(methoxy)-aminosulfonamid
4,16 g (0,0268 Mol) 2-Amino-4,6-dimethoxypyrimidin werden in 100 ml wasserfreiem Dichlormethan gelöst und bei 0 °C mit 6,17 g (0,0268 Mol) N-Methylsulfonyl-methoxyaminosulfonylisocyanat versetzt. Nach 18 Stunden Rühren bei Raumtemperatur wird 2 Stunden auf Rückfluß erhitzt, anschließend wird mit 0,5 n Salzsäure extrahiert. Nach dem Trocknen über Natriumsulfat wird das Produkt bei 0 °C mit n-Heptan ausgefällt. Man erhält 10,02 g (97,1 % d.Th.) an N-[(4,6-Dimethoxy-pyrimidin-2-yl)-aminocarbonyl]-N'-(methylsulfonyl)-N'-(methoxy)-aminosulfonamid vom Schmelzpunkt 142-144 °C.

Auf gleiche Weise werden auch die übrigen in der folgenden Tabelle 2 aufgeführten erfindungsgemäßen Sulfonylharnstoffe der allgemeinen Formel (I), worin A eine direkte Bindung bedeutet, hergestellt.

Tabelle 2

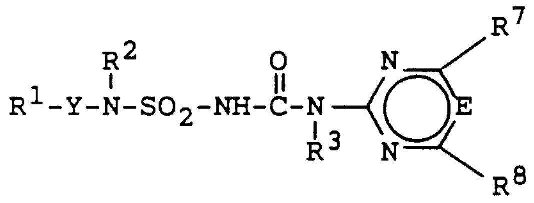

| Bsp Nr. | $-Y-R^1$ | $R^2$ | $R^3$ | $R^7$ | $R^8$ | E | Fp. (°C) |
|---|---|---|---|---|---|---|---|
| 301 | $-SO_2-C_6H_5$ | $OC_2H_5$ | H | $CH_3$ | $CH_3$ | CH | |
| 302 | " | " | H | $OCH_3$ | $CH_3$ | CH | |
| 303 | " | " | H | $OCH_3$ | $OCH_3$ | CH | |
| 304 | " | " | H | $OCH_3$ | Cl | CH | |
| 305 | " | " | H | $OCHF_2$ | $CH_3$ | CH | |
| 306 | " | " | H | $OCHF_2$ | $CF_3$ | CH | |
| 307 | " | " | H | $OCHF_2$ | $OCHF_2$ | CH | |
| 308 | " | " | H | $CH_3$ | Cl | CH | |
| 309 | " | " | H | $OCH_3$ | Br | CH | |
| 310 | $SO_2-CH(CH_3)_2$ | $OCH_3$ | H | $OCH_3$ | $NHCH_3$ | CH | |
| 311 | " | " | H | $OCHF_2$ | $OCH_3$ | CH | |
| 312 | " | " | H | $CH_3$ | $CH_3$ | CH | |
| 313 | " | " | H | $OCH_3$ | $CH_3$ | CH | |
| 314 | " | " | H | $OCH_3$ | $OCH_3$ | CH | 132-133 |
| 315 | " | " | H | $OCH_3$ | Cl | CH | |
| 316 | " | " | H | $OCHF_2$ | $CH_3$ | CH | |
| 317 | " | " | H | $OCHF_2$ | $CF_3$ | CH | |
| 318 | " | " | H | $OCHF_2$ | $OCHF_2$ | CH | |
| 319 | " | " | H | $CH_3$ | Cl | CH | |
| 320 | " | " | H | $OCH_3$ | Br | CH | |
| 321 | " | " | H | $OCH_3$ | $NHCH_3$ | N | |
| 322 | " | " | H | $OCH_3$ | $OCH_3$ | N | |
| 323 | $-SO_2-C_6H_{11}$ | " | H | $OCH_3$ | $OCH_3$ | N | |
| 324 | " | " | H | $OCHF_2$ | $OCHF_2$ | N | |
| 325 | " | " | H | $CH_3$ | $CH_3$ | N | |

Tabelle 2 (Fortsetzung)

| Bsp Nr. | -Y-$R^1$ | $R^2$ | $R^3$ | $R^7$ | $R^8$ | E | Fp. (°C) |
|---|---|---|---|---|---|---|---|
| 326 | $-SO_2-CH_3$ | $OCH_3$ | $CH_3$ | $OCH_3$ | $OCH_3$ | N | |
| 327 | " | " | $CH_3$ | $CH_3$ | $OCHF_2$ | N | |
| 328 | " | " | H | $OCH_3$ | $OCH_3$ | N | |
| 329 | " | " | $CH_3$ | $OCH_3$ | $CF_3$ | N | |
| 330 | $SO_2-CH_2C_6H_5$ | " | H | $CH_3$ | $CH_3$ | N | |
| 331 | " | " | H | $OCH_3$ | $CH_3$ | N | |
| 332 | " | " | H | $OCH_3$ | $OCH_3$ | N | |
| 333 | " | " | H | $OCH_3$ | Cl | N | |
| 334 | " | " | H | $OCHF_2$ | $CH_3$ | N | |
| 335 | " | " | H | $OCHF_2$ | $CF_3$ | N | |
| 336 | " | " | H | $OCHF_2$ | $OCHF_2$ | N | |
| 337 | " | " | H | $CH_3$ | Cl | N | |
| 338 | " | " | H | $OCH_3$ | Br | N | |
| 339 | " | " | H | $OCH_3$ | $NHCH_3$ | N | |
| 340 | $SO_2-C_2H_5$ | $OCH(CH_3)_2$ | H | $OCHF_2$ | $CF_3$ | CH | |
| 341 | " | " | H | $OCHF_2$ | $OCHF_2$ | CH | |
| 342 | " | " | H | $CH_3$ | Cl | CH | |
| 343 | " | " | H | $OCH_3$ | Br | CH | |
| 344 | " | " | H | $OCH_3$ | $NHCH_3$ | CH | |
| 345 | " | " | H | $OCHF_2$ | $OCH_3$ | CH | |
| 346 | " | $OCH_3$ | H | $CH_3$ | $CH_3$ | CH | |
| 347 | " | " | H | $OCH_3$ | $CH_3$ | CH | 85-90 |
| 348 | " | " | H | $OCH_3$ | $OCH_3$ | CH | 159-160 |
| 349 | " | " | H | $OCH_3$ | Cl | CH | |
| 350 | " | $OCH_2CH_2CH_3$ | H | $CH_3$ | $CH_3$ | CH | |
| 351 | " | " | H | $OCH_3$ | $CH_3$ | CH | |
| 352 | " | " | H | $OCH_3$ | $OCH_3$ | CH | |
| 353 | " | " | H | $OCH_3$ | Cl | CH | |
| 354 | " | " | H | $OCHF_2$ | $CH_3$ | CH | |
| 355 | " | " | H | $OCHF_2$ | $CF_3$ | CH | |
| 356 | " | " | H | $OCHF_2$ | $OCHF_2$ | CH | |

Tabelle 2 (Fortsetzung)

| Bsp Nr. | -Y-$R^1$ | $R^2$ | $R^3$ | $R^7$ | $R^8$ | E | Fp. (°C) |
|---|---|---|---|---|---|---|---|
| 357 | $SO_2C_2H_5$ | $OCH_2CH_2CH_3$ | H | $CH_3$ | Cl | CH | |
| 358 | " | " | H | $OCH_3$ | Br | CH | |
| 359 | " | " | H | $OCH_3$ | $NHCH_3$ | CH | |
| 360 | " | " | H | $OCHF_2$ | $OCH_3$ | CH | |
| 361 | " | $OCH(CH_3)_2$ | H | $CH_3$ | $CH_3$ | CH | |
| 362 | " | " | H | $OCH_3$ | $CH_3$ | CH | |
| 363 | " | " | H | $OCH_3$ | $OCH_3$ | CH | |
| 364 | " | " | H | $OCH_3$ | Cl | CH | |
| 365 | " | " | H | $OCHF_2$ | $CH_3$ | CH | |
| 366 | " | $O(CH_2)_3CH_3$ | H | $OCH_3$ | $NHCH_3$ | CH | |
| 367 | " | " | H | $OCHF_2$ | $OCH_3$ | CH | |
| 368 | " | $OCH_3$ | H | $OCHF_2$ | $CH_3$ | CH | |
| 369 | " | " | H | $OCHF_2$ | $CF_3$ | CH | |
| 370 | " | " | H | $OCHF_2$ | $OCHF_2$ | CH | |
| 371 | " | " | H | $CH_3$ | Cl | CH | |
| 372 | " | " | H | $OCH_3$ | Br | CH | |
| 373 | " | " | H | $OCH_3$ | $NHCH_3$ | CH | |
| 374 | " | " | H | $OCHF_2$ | $OCH_3$ | CH | |
| 375 | " | $O(CH_2)_3CH_3$ | H | $CH_3$ | $CH_3$ | CH | |
| 376 | " | " | H | $OCH_3$ | $CH_3$ | CH | |
| 377 | " | " | H | $OCH_3$ | $OCH_3$ | CH | |
| 378 | " | " | H | $OCH_3$ | Cl | CH | |
| 379 | " | " | H | $OCHF_2$ | $CH_3$ | CH | |
| 380 | " | " | H | $OCHF_2$ | $CF_3$ | CH | |
| 381 | " | " | H | $OCHF_2$ | $OCHF_2$ | CH | |
| 382 | " | " | H | $CH_3$ | Cl | CH | |
| 383 | " | " | H | $OCH_3$ | Br | CH | |
| 384 | " | $OC_6H_{11}$ | H | $CH_3$ | $CH_3$ | CH | |
| 385 | " | " | H | $OCH_3$ | $CH_3$ | CH | |
| 386 | " | " | H | $OCH_3$ | $OCH_3$ | CH | |

Tabelle 2 (Fortsetzung)

| Bsp Nr. | $-Y-R^1$ | $R^2$ | $R^3$ | $R^7$ | $R^8$ | E | Fp. (°C) |
|---|---|---|---|---|---|---|---|
| 387 | $-SO_2C_2H_5$ | $OC_6H_{11}$ | H | $OCH_3$ | Cl | CH | |
| 388 | " | " | H | $OCHF_2$ | $CH_3$ | CH | |
| 389 | " | " | H | $OCHF_2$ | $CH_3$ | CH | |
| 390 | " | " | H | $OCHF_2$ | $OCHF_2$ | CH | |
| 391 | " | " | H | $CH_3$ | Cl | CH | |
| 392 | " | " | H | $OCH_3$ | Br | CH | |
| 393 | " | " | H | $OCH_3$ | $NHCH_3$ | CH | |
| 394 | " | " | H | $OCHF_2$ | $OCH_3$ | CH | |
| 395 | $-SO_2CH_3$ | $OC_3H_7$ | H | $CH_3$ | $CH_3$ | CH | |
| 396 | " | " | H | $OCH_3$ | $CH_3$ | CH | |
| 397 | " | " | H | $OCH_3$ | $OCH_3$ | CH | |
| 398 | " | " | H | $OCH_3$ | Cl | CH | |
| 399 | " | " | H | $OCHF_2$ | $CH_3$ | CH | |
| 400 | " | " | H | $OCHF_2$ | $CF_3$ | CH | |
| 401 | " | " | H | $OCHF_2$ | $OCHF_2$ | CH | |
| 402 | " | " | H | $CH_3$ | Cl | CH | |
| 403 | " | " | H | $OCH_3$ | Br | CH | |
| 404 | " | " | H | $OCH_3$ | $NHCH_3$ | CH | |
| 405 | " | " | H | $OCHF_2$ | $OCH_3$ | CH | |
| 406 | " | $OCH_2CH_3$ | H | $CH_3$ | $CH_3$ | CH | |
| 407 | " | " | H | $OCH_3$ | $CH_3$ | CH | |
| 408 | " | " | H | $OCH_3$ | $OCH_3$ | CH | |
| 409 | " | " | H | $OCH_3$ | Cl | CH | |
| 410 | " | " | H | $OCHF_2$ | $CH_3$ | CH | |
| 411 | " | " | H | $OCHF_2$ | $CF_3$ | CH | |
| 412 | " | " | H | $OCHF_2$ | $OCHF_2$ | CH | |
| 413 | " | " | H | $CH_3$ | Cl | CH | |
| 414 | " | " | H | $OCH_3$ | Br | CH | |
| 415 | " | " | H | $OCH_3$ | $NHCH_3$ | CH | |
| 416 | " | " | H | $OCHF_2$ | $OCH_3$ | CH | |

**Tabelle 2** (Fortsetzung)

| Bsp Nr. | $-Y-R^1$ | $R^2$ | $R^3$ | $R^7$ | $R^8$ | E | Fp. (°C) |
|---|---|---|---|---|---|---|---|
| 417 | $-SO_2CH_2CH_3$ | $OC_6H_5$ | H | $CH_3$ | $CH_3$ | CH | |
| 418 | " | " | H | $CH_3$ | $OCH_3$ | CH | |
| 419 | " | " | H | $OCH_3$ | $OCH_3$ | CH | |
| 420 | " | " | H | $OCH_3$ | $OCH_3$ | N | |
| 421 | " | " | H | $CH_3$ | $OCH_3$ | N | |
| 422 | " | " | H | $OCHF_2$ | $OCHF_2$ | CH | |
| 423 | $SO_2C_6H_5$ | $OCH_3$ | H | $CH_3$ | $CH_3$ | CH | |
| 424 | " | " | H | $CH_3$ | $OCH_3$ | CH | |
| 425 | " | " | H | $OCH_3$ | $OCH_3$ | CH | 158-159 |
| 426 | " | " | H | $OCH_3$ | $OCH_3$ | N | |
| 427 | " | " | H | $CH_3$ | $OCH_3$ | N | |
| 428 | " | " | H | $OCHF_2$ | $OCH_3$ | CH | |
| 429 | $SO_2N(CH_3)_2$ | " | H | $CH_3$ | $CH_3$ | CH | |
| 430 | " | " | H | $CH_3$ | $OCH_3$ | CH | |
| 431 | " | " | H | $OCH_3$ | Cl | CH | |
| 432 | " | " | H | $OCH_3$ | $OCH_3$ | N | |
| 433 | " | " | H | $CH_3$ | $OCH_3$ | N | |
| 434 | " | " | H | $OCHF_2$ | $OCHF_2$ | CH | |
| 435 | " | $OC_2H_5$ | H | $CH_3$ | $CH_3$ | CH | |
| 436 | " | " | H | $CH_3$ | $OCH_3$ | CH | |
| 437 | " | " | H | $OCH_3$ | $OCH_3$ | CH | |
| 438 | " | " | H | $OCH_3$ | $OCH_3$ | N | |
| 439 | " | " | H | $CH_3$ | $OCH_3$ | N | |
| 440 | " | " | H | $OCHF_2$ | $OCHF_2$ | CH | |
| 441 | " | $OC_3H_7(n)$ | H | $OCH_3$ | $OCH_3$ | CH | |
| 442 | " | " | H | $OCH_3$ | $OCH_3$ | N | |
| 443 | " | " | H | $CH_3$ | $OCH_3$ | CH | |
| 444 | " | " | H | $CH_3$ | $OCH_3$ | N | |
| 445 | " | $OC_4H_9(n)$ | H | $OCH_3$ | $OCH_3$ | CH | |
| 446 | " | " | H | $OCH_3$ | Cl | CH | |

**Tabelle 2** (Fortsetzung)

| Bsp Nr. | -Y-$R^1$ | $R^2$ | $R^3$ | $R^7$ | $R^8$ | E | Fp. (°C) |
|---|---|---|---|---|---|---|---|
| 447 | $SO_2NHCH_3$ | $OCH_3$ | H | $OCH_3$ | $OCH_3$ | CH | |
| 448 | " | " | H | $OCH_3$ | Cl | CH | |
| 449 | " | " | H | $OCH_3$ | $CH_3$ | CH | |
| 450 | " | " | H | $CH_3$ | $CH_3$ | CH | |
| 451 | " | " | H | $CH_3$ | $OCH_3$ | N | |
| 452 | " | " | H | $OCH_3$ | $OCH_3$ | N | |
| 453 | $SO_2NHC_2H_5$ | " | H | $OCH_3$ | $OCH_3$ | CH | |
| 454 | " | " | H | $OCH_3$ | Cl | CH | |
| 455 | " | " | H | $OCH_3$ | $CH_3$ | N | |
| 456 | " | " | H | $OCH_3$ | $OCH_3$ | N | |
| 457 | $SO_2N$ 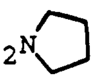 | " | H | $OCH_3$ | $OCH_3$ | CH | |
| 458 | " | " | $CH_3$ | $OCH_3$ | $OCH_3$ | CH | |
| 459 | " | " | H | $OCH_3$ | $CH_3$ | CH | |
| 460 | $SO_2N$ 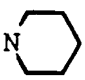 | " | H | $OCH_3$ | $CH_3$ | CH | |
| 461 | " | " | H | $OCH_3$ | $OCH_3$ | CH | |
| 462 | $SO_2C_2H_5$ | " | $CH_3$ | $OCH_3$ | $OCH_3$ | CH | |
| 463 | " | " | $CH_3$ | $OCH_3$ | $CH_3$ | N | |
| 464 | $SO_2N(CH_3)_2$ | " | $CH_3$ | $OCH_3$ | $OCH_3$ | CH | |
| 465 | " | " | $CH_3$ | $OCH_3$ | $CH_3$ | N | |
| 466 | $SO_2NHCH_3$ | " | $CH_3$ | $OCH_3$ | $OCH_3$ | CH | |
| 467 | $SOCH(CH_3)_2$ | $OC_2H_5$ | H | $OCH_3$ | $OCH_3$ | CH | |
| 468 | " | " | H | $OCH_3$ | $CH_3$ | CH | |
| 469 | " | " | H | Cl | $CH_3$ | CH | |
| 470 | " | " | H | $OCH_3$ | $OCH_3$ | N | |
| 471 | $SO_2CH_3$ | $OCH_3$ | H | $CH_3$ | $CH_3$ | CH | 83-85 |
| 472 | " | " | H | $OCH_3$ | $CH_3$ | CH | 81-83 |
| 473 | " | " | H | $OCH_3$ | $OCH_3$ | CH | 142-144 |

Tabelle 2 (Fortsetzung)

| Bsp Nr. | -Y-$R^1$ | $R^2$ | $R^3$ | $R^7$ | $R^8$ | E | Fp. (°C) |
|---|---|---|---|---|---|---|---|
| 474 | $SO_2CH_3$ | $OCH_3$ | H | $OCH_3$ | Cl | CH | |
| 475 | " | " | H | $OCHF_2$ | $CH_3$ | CH | |
| 476 | " | " | H | $OCHF_2$ | $CF_3$ | CH | |
| 477 | " | " | H | $OCHF_2$ | $OCHF_2$ | CH | |
| 478 | " | " | H | $CH_3$ | Cl | CH | |
| 479 | " | " | H | $OCH_3$ | Br | CH | |
| 480 | " | " | H | $OCH_3$ | $NHCH_3$ | CH | |
| 481 | " | " | H | $OCHF_2$ | $OCH_3$ | CH | |
| 482 | $SO_2(n)C_3H_7$ | " | H | $CH_3$ | $CH_3$ | CH | 115-118 |
| 483 | " | " | H | $OCH_3$ | $CH_3$ | CH | |
| 484 | " | " | H | $OCH_3$ | $OCH_3$ | CH | 163-165 |
| 485 | " | " | H | $OCH_3$ | Cl | CH | 143-145 |
| 486 | " | " | H | $OCHF_2$ | $OCHF_2$ | CH | |
| 487 | $SO_2CH_2CH_3$ | $OCH_2CH_3$ | H | $CH_3$ | $CH_3$ | CH | 148-150 |
| 488 | " | " | H | $OCH_3$ | $CH_3$ | CH | 142-144 |
| 489 | " | " | H | $OCH_3$ | $OCH_3$ | CH | 145-147 |
| 490 | " | " | H | $OCH_3$ | Cl | CH | |

## BIOLOGISCHE BEISPIELE

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

### 1. Unkrautwirkung im Vorauflauf

Samen bzw. Rhizomstücke von mono- und dikotylen Unkrautpflanzen wurden in Plostiktöpfen in sandiger Lehmerde ausgelegt und mit Erde abgedeckt. Die in Form von benetzbaren Pulver oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte in Tabelle 3 zeigen, weisen die erfindungsgemäßen Verbindungen eine gute herbizide Vorauflaufwirksamkeit gegen ein breites Spektrum von Ungräsern und

Unkräutern auf.

Tabelle 3

| Vorauflaufwirkung der erfindungsgemäßen Verbindungen der Formel (I) | | | | | |
|---|---|---|---|---|---|
| Beispiel-Nr. | Dosis (kg a.i./ha) | herbizide Wirkung | | | |
| | | STM | CRS | SIA | LOM |
| 24 | 0,3 | 5 | 5 | 1 | 2 |
| 25 | 0,3 | 5 | 5 | 4 | 4 |
| 105 | 0,3 | 5 | 5 | 5 | 3 |
| 106 | 0,3 | 5 | 5 | 5 | 4 |
| | 0,08 | 5 | 5 | 5 | 4 |
| 107 | 0,3 | 4 | 3 | 3 | 1 |
| 165 | 0,3 | 5 | 2 | 2 | 2 |
| 166 | 0,3 | 5 | 3 | 4 | 3 |
| 225 | 0,3 | 5 | 5 | 4 | 4 |
| 226 | 0,3 | 5 | 3 | 1 | 1 |
| 231 | 0,3 | 5 | 5 | 5 | 5 |
| 232 | 0,3 | 5 | 5 | 5 | 5 |
| 237 | 0,3 | 5 | 5 | 5 | 5 |
| 238 | 0,3 | 4 | 4 | 1 | 2 |
| 347 | 0,3 | 5 | 5 | 5 | 5 |
| 348 | 0,3 | 5 | 5 | 5 | 5 |
| 425 | 0,3 | 2 | 2 | 3 | 1 |
| 471 | 0,3 | 2 | 4 | 5 | 2 |
| 472 | 0,3 | 5 | 5 | 5 | 3 |
| 473 | 0,3 | 5 | 5 | 5 | 5 |
| 482 | 0,3 | 1 | 4 | 5 | 1 |
| 484 | 0,3 | 5 | 5 | 5 | 4 |
| 485 | 0,3 | 2 | 4 | 5 | 2 |
| 488 | 0,3 | 5 | 5 | 5 | 5 |
| STM = Stellaria media<br>CRS = Chrysanthemum segetum<br>SIA = Sinapis alba<br>LOM = Lolium multiflorum | | | | | |

**2. Unkrautwirkung im Nachauflauf**

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 4).

Tabelle 4

| Nachauflaufwirkung der erfindungsgemäßen Verbindungen der Formel (I) | | | | | | |
|---|---|---|---|---|---|---|
| Beispiel-Nr. | Dosis (kg a.i./ha) | herbizide Wirkung | | | |
| | | STM | CRS | SIA | LOM |
| 24 | 0,3 | 4 | 2 | 2 | 0 |
| 25 | 0,3 | 5 | 5 | 4 | 0 |
| 105 | 0,3 | 5 | 4 | 4 | 1 |
| 106 | 0,3 | 5 | 5 | 5 | 1 |
| | 0,08 | 5 | 5 | 5 | 1 |
| 107 | 0,3 | 5 | 2 | 2 | 0 |
| 165 | 0,3 | 5 | 2 | 3 | 0 |
| 166 | 0,3 | 5 | 4 | 4 | 2 |
| 225 | 0,3 | 5 | 4 | 4 | 0 |
| 226 | 0,3 | 4 | 1 | 4 | 1 |
| 231 | 0,3 | 5 | 5 | 1 | 2 |
| 232 | 0,3 | 5 | 3 | 4 | 3 |
| 237 | 0,3 | 5 | 4 | 5 | 3 |
| 238 | 0,3 | 5 | 2 | 3 | 0 |
| 347 | 0,3 | 5 | 5 | 5 | 4 |
| 348 | 0,3 | 5 | 5 | 5 | 4 |
| 425 | 0,3 | 3 | 2 | 4 | 1 |
| 472 | 0,3 | 5 | 4 | 5 | 3 |
| 473 | 0,3 | 5 | 5 | 5 | 2 |
| 482 | 0,3 | 4 | 5 | 5 | 1 |
| 484 | 0,3 | 4 | 4 | 5 | 3 |
| 485 | 0,3 | 1 | 2 | 5 | 1 |
| 488 | 0,3 | 4 | 5 | 5 | 3 |
| Abkürzungen: s. Tabelle 3 | | | | | |

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschtem Pflanzenwuchs in landwirtschaftlichen Kulturen auf.

### 4. Wuchshemmung an Getreide

In Schalenversuchen im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnass gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet, wobei 100 %

den Stillstand des Wachstums und 0 % ein Wachstum entsprechend den unbehandelten Kontrollpflanzen bedeutet. Es zeigte sich, daß die Verbindungen sehr gute wachstumsregulierende Eigenschaften besitzen.

**Patentansprüche**

**1.** Verbindungen der allgemeinen Formel (I),

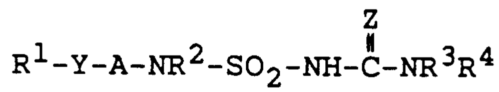

(I)

worin

A — eine direkte Bindung oder einen gesättigten oder ungesättigten, unverzweigten oder verzweigten $C_1$-$C_{10}$-Kohlenwasserstoffrest,

$R^1$ — $C_1$-$C_8$-Alkyl, $C_2$-$C_8$-Alkenyl, $C_2$-$C_8$-Alkinyl, $C_3$-$C_8$-Cycloalkyl, $C_5$-$C_8$-Cycloalkenyl oder einen der vorstehenden fünf Reste, der ein- oder mehrfach durch Halogen oder durch solche Reste substituiert ist, die aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, $C_3$-$C_6$-Cycloalkyl, einem Rest eines drei- bis sechsgliedrigen gesättigten Heterozyklus mit einem Sauerstoffatom im Ring, Furyl, Phenyl und einem Phenylrest, der ein- oder mehrfach durch Reste aus der Gruppe aus Halogen, CN, $C_1$-$C_4$-Alkyl, $C_1$-$C_4$-Alkoxy, $C_1$-$C_4$-Halogenalkyl, ($C_1$-$C_4$-Alkoxy)-carbonyl und Nitro substituiert ist, ausgewählt sind, oder
Phenyl oder einen Phenylrest, der ein- oder mehrfach durch Reste aus der Gruppe bestehend aus Halogen, $C_1$-$C_4$-Alkyl, $C_1$-$C_4$-Alkoxy, $C_1$-$C_4$-Halogenalkyl, ($C_1$-$C_4$-Alkoxy)-carbonyl und Nitro substituiert ist, oder einen Rest der Formel
$-NR^5R^6$, wobei $R^5$ und $R^6$ unabhängig voneinander Wasserstoff oder $C_1$-$C_8$-Alkyl oder einer der Reste $R^5$ bzw. $R^6$ $C_1$-$C_4$-Alkoxy bedeuten oder $R^5$ und $R^6$ gemeinsam eine Alkylenkette $-(CH_2)_n-$ mit $n = 2$ bis 7 bilden;

Y — S, SO oder $SO_2$,

$R^2$ — $C_1$-$C_8$-Alkyloxy, $C_2$-$C_8$-Alkenyloxy, $C_2$-$C_8$-Alkinyloxy oder einen der vorstehenden 3 Reste, der ein- oder mehrfach durch Halogen oder durch Reste aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, ($C_1$-$C_6$-Alkoxy)-carbonyl, Phenoxycarbonyl, Benzyloxycarbonyl und Phenyl substituiert ist,
$C_3$-$C_8$-Cycloalkyloxy, das unsubstituiert ist oder ein-oder mehrfach durch Halogen oder ein- oder zweifach durch $C_1$-$C_4$-Alkoxy oder $C_1$-$C_4$-Alkylthio substituiert ist, $C_5$-$C_8$-Cycloalkenyloxy, Cyclopropylmethyloxy, Epoxypropyloxy, Furfuryloxy, Tetrahydrofurfuryloxy, Phenoxy-$C_1$-$C_6$-alkyloxy, Phenoxy oder einen der letzten zwei vorstehenden Reste, der im Phenylring durch Halogen, $C_1$-$C_4$-Alkyl, $C_1$-$C_4$-Alkoxy oder Nitro substituiert ist,

$R^3$ — H, $C_1$-$C_8$-Alkyl, $C_2$-$C_8$-Alkenyl, $C_2$-$C_8$-Alkinyl oder $C_1$-$C_4$-Alkoxy,

$R^4$ — einen Rest der Formel

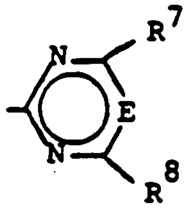

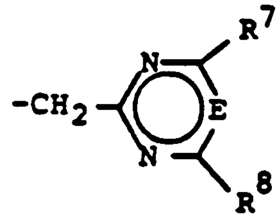

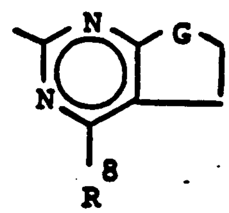

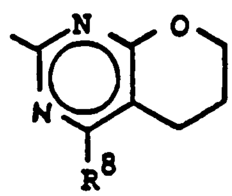

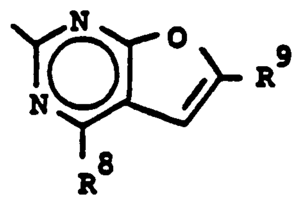

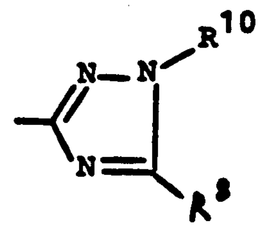

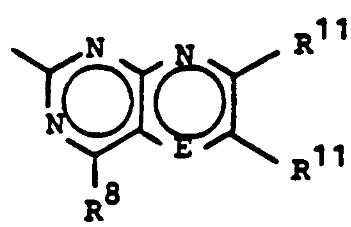

oder

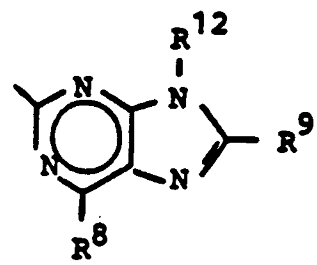

| | |
|---|---|
| $R^7$ und $R^8$ | unabhängig voneinander H, Halogen, $C_1$-$C_6$-Alkyl, $C_1$-$C_6$-Alkoxy, $C_1$-$C_6$-Alkylthio oder einen der letzten drei vorstehenden Reste, der ein- oder mehrfach durch Halogen oder ein- oder zweifach durch $C_1$-$C_4$-Alkoxy oder $C_1$-$C_4$-Alkylthio substituiert ist, oder einen Rest $NR^{13}R^{14}$, $C_3$-$C_6$-Cycloalkyl, $-OCHR^{15}COOR^{16}$, $C_3$-$C_5$-Alkenyl, $C_2$-$C_4$-Alkinyl, $C_3$-$C_5$-Alkenyloxy oder $C_3$-$C_5$-Alkinyloxy, |
| $R^9$ | Wasserstoff oder $C_1$-$C_4$-Alkyl, |
| $R^{10}$ | $C_1$-$C_4$-Alkyl, $-CHF_2$ oder $-CH_2CF_3$, |
| $R^{11}$ | unabhängig voneinander H, $C_1$-$C_4$-Alkyl, $C_1$-$C_4$-Alkoxy oder Halogen, |
| $R^{12}$ | Wasserstoff, $C_1$-$C_4$-Alkyl, $CHF_2$ oder $CH_2CF_3$, |
| $R^{13}$ und $R^{14}$ | unabhängig voneinander H, $C_1$-$C_4$-Alkyl, $C_2$-$C_4$-Alkenyl oder $C_3$-$C_4$-Alkinyl, |
| $R^{15}$ | Wasserstoff oder $C_1$-$C_4$-Alkyl, |
| $R^{16}$ | Wasserstoff oder $C_1$-$C_4$-Alkyl, |
| E | CH oder N, |
| G | $CH_2$ oder O und |
| Z | O oder S bedeuten, sowie ihre Salze. |

**2.** Verbindungen der Formel (I) und ihre Salze nach Anspruch 1, dadurch gekennzeichnet, daß A eine direkte Bindung oder einen divalenten Rest der Formel $CH_2CH_2$, CRR′, $CH_2CHR$ oder $CH_2CRR'$ bedeutet, wobei R und R′ unabhängig voneinander $C_1$-$C_4$-Alkyl oder $C_2$-$C_4$-Alkenyl bedeutet.

**3.** Verbindungen der Formel (I) und ihre Salze nach Anspruch 1 oder 2, dadurch gekennzeichnet, daß $R^1$ $C_1$-$C_4$-Alkyl, einen $C_1$-$C_4$-Alkylrest, der ein- oder mehrfach durch Halogen oder ein- oder zweifach durch $C_1$-$C_4$-Alkoxy, $C_2$-$C_3$-Alkenyloxy, $C_2$-$C_3$-Alkinyloxy, $C_1$-$C_4$-Alkylthio, $C_1$-$C_4$-Alkylsulfinyl, $C_1$-$C_4$-Alkylsulfonyl, Phenyl oder einen Phenylrest, der ein- bis dreifach durch Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, substituiert ist, oder $C_3$-$C_8$-Cycloalkyl oder einen $C_3$-$C_8$-Cycloalkylrest, der ein-oder mehrfach durch Halogen oder ein- bis zweifach durch $C_1$-$C_4$-Alkoxy oder $C_1$-$C_4$-Alkylthio substituiert ist, oder $C_5$-$C_8$-Cycloalkenyl, Cyclopropylmethyl, Epoxypropyl, Furfuryl, Tetrahydrofurfuryl, Benzyl, Phenyl, oder einen Benzyl- oder

Phenylrest, der im Phenylring durch einen oder mehrere Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, einen Rest der Formel $NR^5R^6$, worin $R^5$ $C_1$-$C_4$-Alkyl und $R^6$ Wasserstoff oder $C_1$-$C_4$-Alkyl bedeuten oder $R^5$ und $R^6$ gemeinsam eine Alkylenkette $-(CH_2)_n-$ mit n = 4 oder 5 bilden, bedeutet.

**4.** Verbindungen der Formel (I) und ihre Salze nach einem oder mehreren der Ansprüche 1 bis 3, dadurch gekennzeichnet, daß $R^2$ $C_1$-$C_4$-Alkyloxy, einen $C_1$-$C_4$-Alkyloxyrest, der ein- oder mehrfach durch Halogen oder ein- oder zweifach durch $C_1$-$C_4$-Alkoxy, $C_2$-$C_4$-Alkenyloxy, Propargyloxy, $C_1$-$C_4$-Alkylthio, $C_1$-$C_4$-Alkylsulfinyl, $C_1$-$C_4$-Alkylsulfonyl, ($C_1$-$C_4$-Alkoxy)-carbonyl, Phenoxycarbonyl, Benzyloxycarbonyl oder Phenyl substituiert ist, oder $C_3$-$C_8$-Cycloalkyloxy bedeutet.

**5.** Verbindungen der Formel (I) und ihre Salze nach einem oder mehreren der Ansprüche 1 bis 4, dadurch gekennzeichnet, daß $R^4$ einen Rest der Formel

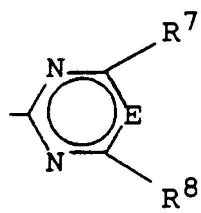

und

| | |
|---|---|
| $R^7$ und $R^8$ | unabhängig voneinander Halogen, $C_1$-$C_4$-Alkyl, $C_1$-$C_4$-Alkoxy, $C_1$-$C_4$-Alkylthio oder einen der letzten drei vorstehenden Reste, die ein- oder mehrfach durch Halogen oder ein- oder zweifach durch $C_1$-$C_4$-Alkoxy, $C_1$-$C_4$-Alkylthio substituiert sind, oder einen Rest $NR^{13}R^{14}$, $C_3$-$C_6$-Cycloalkyl, $-OCHR^{15}COOR^{16}$, Allyl, Propargyl, Allyloxy oder Propargyloxy bedeuten, |
| $R^{13}$ und $R^{14}$ | unabhängig voneinander H oder $C_1$-$C_4$-Alkyl, |
| $R^{15}$ | H oder $C_1$-$C_4$-Alkyl, |
| $R^{16}$ | $C_1$-$C_4$-Alkyl und |
| E | CH oder N bedeuten. |

**6.** Verbindungen der Formel (I) und ihre Salze nach einem oder mehreren der Ansprüche 1 bis 5, dadurch gekennzeichnet, daß

| | |
|---|---|
| A | eine direkte Bindung, |
| $R^1$ | $C_1$-$C_4$-Alkyl, einen $C_1$-$C_4$-Alkylrest, der ein- oder mehrfach durch Halogen oder ein- bis zweifach durch $C_1$-$C_4$-Alkoxy substituiert ist, einen Rest der Formel $NR^5R^6$, worin $R^5$ $C_1$-$C_4$-Alkyl und $R^6$ Wasserstoff oder $C_1$-$C_4$-Alkyl bedeuten oder $R^5$ und $R^6$ gemeinsam eine Alkylenkette $-(CH_2)_n-$mit n = 4 oder 5 bilden, |
| $R^2$ | $C_1$-$C_4$-Alkoxy, das unsubstituiert ist oder ein- oder mehrfach durch Halogen oder durch ($C_1$-$C_4$-Alkoxy)-carbonyl, Phenyloxycarbonyl, Benzyloxycarbonyl oder Phenyl substituiert ist, |
| $R^3$ | H, $C_1$-$C_4$-Alkyl oder Allyl, |
| $R^4$ | einen Rest der Formel |

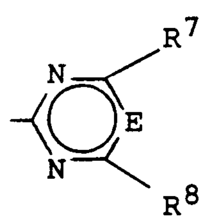

| | |
|---|---|
| $R^7$ und $R^8$ | unabhängig voneinander Halogen, $C_1$-$C_4$-Alkyl, $C_1$-$C_4$-Alkoxy oder einen der letzten |

beiden vorstehenden Reste, der halogeniert ist,

E CH oder N und

Z ein Sauerstoffatom

bedeuten.

**7.** Verbindungen der Formel (I) und ihre Salze nach einem oder mehreren der Ansprüche 1 bis 5, dadurch gekennzeichnet, daß

A einen Rest der Formel $-CH_2-CH_2-$,

$R^1$ $C_1$-$C_4$-Alkyl, einen $C_1$-$C_4$-Alkylrest, der ein- oder mehrfach durch Halogen oder ein- bis zweifach durch $C_1$-$C_4$-Alkoxy substituiert ist, $C_3$-$C_8$-Cycloalkyl, das ein- oder mehrfach durch Halogen substituiert ist oder unsubstituiert ist, Benzyl, Phenyl oder einen Benzyl- oder Phenylrest, der im Phenylring ein- oder mehrfach durch Halogen, $C_1$-$C_4$-Alkyl, $C_1$-$C_4$-Alkoxy, $CF_3$, ($C_1$-$C_4$-Alkoxy)-carbonyl oder Nitro substituiert ist,

$R^2$ $C_1$-$C_4$-Alkoxy, das unsubstituiert ist oder ein- oder mehrfach durch Halogen oder durch ($C_1$-$C_4$-Alkoxy)-carbonyl, Phenyloxycarbonyl, Benzyloxycarbonyl oder Phenyl substituiert ist,

$R^3$ H, $C_1$-$C_4$-Alkyl oder Allyl,

$R^4$ einen Rest der Formel

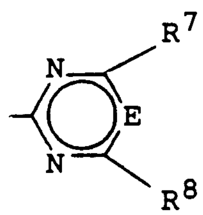

$R^7$ und $R^8$ unabhängig voneinander Halogen, $C_1$-$C_4$-Alkyl, $C_1$-$C_4$-Alkoxy oder einen der letzten beiden vorstehenden Reste, der halogeniert ist,

E CH oder N und

Z O oder S bedeuten.

**8.** Verfahren zur Herstellung von Verbindungen der allgemeinen Formel (I) oder deren Salzen nach Anspruch 1, dadurch gekennzeichnet, daß man

(a) eine Verbindung der Formel (II)

$R^1$-Y-A-$NR^2$-$SO_2$-N = C = Z (II),

mit einer Verbindung der Formel (III)

H-$NR^3R^4$ (III),

umsetzt, wobei in den Formeln (II) und (III) A, Y, Z, $R^1$, $R^2$, $R^3$ und $R^4$ die bei Formel (I) angegebenen Bedeutungen haben, oder

(b) eine Verbindung der Formel (IV)

$R^1$-Y-A-$NR^2$-$SO_2$-$NH_2$ (IV)

mit einem Carbamat bzw. Thiocarbamat der Formel (V)

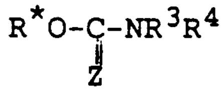

(V)

umsetzt,
wobei in den Formeln (IV) und (V) $R^1$, $R^2$, $R^3$, $R^4$, A, Y und Z die bei Formel (I) angegebenen Bedeutungen haben und $R^*C_1$-$C_6$-Alkyl, $C_1$-$C_4$-Halogenalkyl, Phenyl oder einen Phenylrest bedeutet, der ein- oder mehrfach durch Halogen, $C_1$-$C_4$-Alkyl oder Nitro substituiert ist, oder
(c) ein Carbamat bzw. Thiocarbamat der Formel (VI)

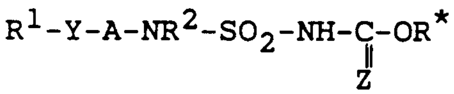
(VI)

mit einer Verbindung der unter (a) genannten Formel (III) umsetzt, wobei $R^1$, $R^2$, $R^*$, Y, A und Z die genannten Bedeutungen haben, oder
(d) eine Verbindung der Formel (VII) oder (VIII)

$R^1$-Y-A-NH-$R^2$ (VII)

$R^1$-Y-A-NH-$R^2$ x HCl (VIII)

mit einer Verbindung der Formel (IX)

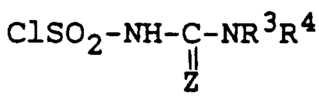
(IX)

umsetzt, wobei in den Formeln (VII) bis (IX) A, Y, Z, $R^1$, $R^2$, $R^3$ und $R^4$ die oben angegebenen Bedeutungen haben.

**9.** Herbizides oder pflanzenwachstumsregulierendes Mittel, dadurch gekennzeichnet, daß es eine Verbindung der Formel (I) oder ihr Salz nach einem oder mehreren der Ansprüche 1 bis 6 oder 1 bis 5 und 7 neben inerten Trägerstoffen enthält.

**10.** Verwendung von Verbindungen der Formel (I) oder deren Salzen nach einem oder mehreren der Ansprüche 1 bis 6 oder 1 bis 5 und 7 als Herbizide oder Pflanzenwachstumsregulatoren.

**11.** Verbindungen der Formel (II)

$R^1$ - Y - A - $NR^2$ - $SO_2$ - N = C = Z (II)

worin
$R^1$, $R^2$, A und Z die bei Formel (I) nach Anspruch 1 definierten Bedeutungen haben.

**12.** Verfahren zur Herstellung der Verbindungen der Formel (II) nach Anspruch 11, dadurch gekennzeichnet, daß man eine Verbindung der Formel (VII)

$R^1$ - Y - A - NH - $R^2$ (VII)

worin
$R^1$-Y-A und $R^2$ die in Anspruch 11 definierte Bedeutung haben, mit Chlorsulfonylisocyanat umsetzt.

**13.** Verbindungen der Formel (VI)

$$R^1\text{-}Y\text{-}A\text{-}NR^2\text{-}SO_2\text{-}NH\text{-}\overset{\overset{\displaystyle Z}{\|}}{C}\text{-}OR^* \qquad (VI)$$

worin
$R^1$, Y, A, $R^2$ und Z die bei Formel (I) nach Anspruch 1 definierten Bedeutungen haben und
$R^*$ $C_1$-$C_6$-Alkyl, $C_1$-$C_4$-Halogenalkyl, Phenyl oder einen Phenylrest, der ein-oder mehrfach durch Halogen, $C_1$-$C_4$-Alkyl oder Nitro substituiert ist, bedeutet.

**14.** Verfahren zur Herstellung der Verbindungen der Formel (VI) nach Anspruch 13, dadurch gekennzeichnet, daß man eine Verbindung der Formel (IV)

$R^1$-Y-A-$NR^2$-$SO_2$-NH (IV)

mit Chlorameisensäureestern der Formel

$$Cl\text{-}\overset{\overset{\displaystyle Z}{\|}}{C}\text{-}OR^*$$

umsetzt, wobei
$R^1$, A, Y, $R^2$ und $R^*$ in den vorstehenden Formeln die in Anspruch 13 definierte Bedeutung haben.

## Claims

**1.** A compound of the general formula (I)

$$R^1\text{-}Y\text{-}A\text{-}NR^2\text{-}SO_2\text{-}NH\text{-}\overset{\overset{\displaystyle Z}{\|}}{C}\text{-}NR^3R^4 \qquad (I)$$

where

| | |
|---|---|
| A | is a direct bond or a saturated or unsaturated, unbranched or branched $C_1$-$C_{10}$-hydrocarbon radical, |
| $R^1$ | is $C_1$-$C_8$-alkyl, $C_2$-$C_8$-alkenyl, $C_2$-$C_8$-alkynyl, $C_3$-$C_8$-cycloalkyl, $C_5$-$C_8$-cycloalkenyl or one of the above five radicals which is monosubstituted or polysubstituted by halogen or by those radicals which are selected from the group comprising $C_1$-$C_6$-alkoxy, $C_2$-$C_6$-alkenyloxy, $C_2$-$C_6$-alkynyloxy, $C_1$-$C_6$-alkylthio, $C_1$-$C_6$-alkylulfinyl, $C_1$-$C_6$-alkylsulfonyl, $C_3$-$C_6$-cycloalkyl, a radical of a three-membered to six-membered saturated heterocycle with one oxygen atom in the ring, furyl, phenyl and a phenyl radical which is monosubstituted or polysubstituted by radicals from the group comprising halogen, CN, $C_1$-$C_4$-alkyl, $C_1$-$C_4$-alkoxy, $C_1$-$C_4$-haloalkyl, or by ($C_1$-$C_4$-alkoxy)carbonyl and nitro, or phenyl or a phenyl radical which is monosubstituted or polysubstituted by radicals from the group embracing halogen, $C_1$-$C_4$-alkyl, $C_1$-$C_4$-alkoxy, $C_1$-$C_4$-haloalkyl, ($C_1$-$C_4$-alkoxy)carbonyl and nitro, or a radical of the formula $-NR^5R^6$, where $R^5$ and $R^6$ independently of one another are hydrogen or $C_1$-$C_8$-alkyl, or one of the radicals $R^5$ or $R^6$ is $C_1$-$C_4$-alkoxy, or $R^5$ and $R^6$ together form an alkylene chain $-(CH_2)_n-$ with n being 2 to 7; |
| Y | is S, SO or $SO_2$, |
| $R^2$ | is $C_1$-$C_8$-alkyloxy, $C_2$-$C_8$-alkenyloxy, $C_2$-$C_8$-alkynyloxy or one of the above 3 radicals which is monosubstituted or polysubstituted by halogen or by radicals from the |

group comprising $C_1$-$C_6$-alkoxy, $C_2$-$C_6$-alkenyloxy, $C_2$-$C_6$-alkynyloxy, $C_1$-$C_6$-alkylthio, $C_1$-$C_6$-alkylsulfinyl, $C_1$-$C_6$-alkylsulfonyl, ($C_1$-$C_6$-alkoxy)-carbonyl, phenoxycarbonyl, benzyloxycarbonyl and phenyl, or $R^2$ is $C_3$-$C_8$-cycloalkyloxy which is unsubstituted or monosubstituted or polysubstituted by halogen, or monosubstituted or disubstituted by $C_1$-$C_4$-alkoxy or $C_1$-$C_4$-alkylthio, $C_5$-$C_8$-cycloalkenyloxy, cyclopropylmethyloxy, epoxypropyloxy, furfuryloxy, tetrahydrofurfuryloxy, phenoxy-$C_1$-$C_6$-alkyloxy, phenoxy or one of the last two abovementioned radicals which is substituted in the phenyl ring by halogen, $C_1$-$C_4$-alkyl, $C_1$-$C_4$-alkoxy or nitro,

$R^3$ is H, $C_1$-$C_8$-alkyl, $C_2$-$C_8$-alkenyl, $C_2$-$C_8$-alkynyl or $C_1$-$C_4$-alkoxy,

$R^4$ is a radical of the formula

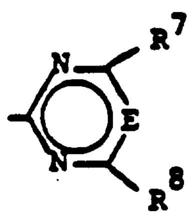
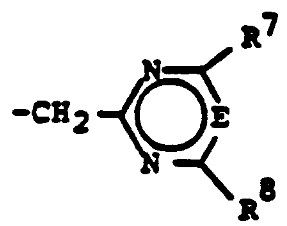
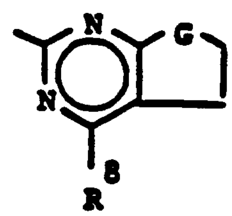
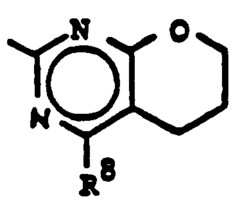
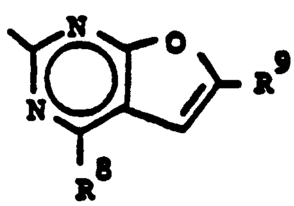
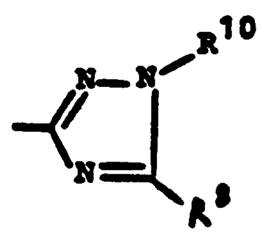
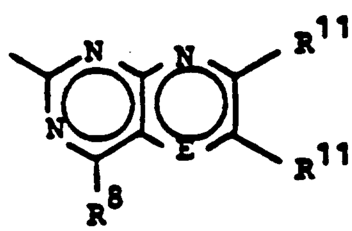
or
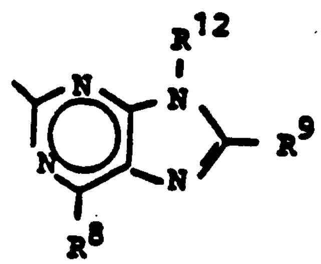

$R^7$ and $R^8$ independently of one another are H, halogen, $C_1$-$C_6$-alkyl, $C_1$-$C_6$-alkoxy, $C_1$-$C_6$-alkylthio or one of the last three abovementioned radicals which is monosubstituted or polysubstituted by halogen or monosubstituted or disubstituted by $C_1$-$C_4$-alkoxy or $C_1$-$C_4$-alkylthio, or are a radical $NR^{13}R^{14}$, $C_3$-$C_6$-cycloalkyl, $-OCHR^{15}COOR^{16}$, $C_3$-$C_5$-alkenyl, $C_2$-$C_4$-alkynyl, $C_3$-$C_5$-alkenyloxy or $C_3$-$C_5$-alkynyloxy,

$R^9$ is hydrogen or $C_1$-$C_4$-alkyl,

$R^{10}$ is $C_1$-$C_4$-alkyl, $-CHF_2$ or $-CH_2CF_3$,

$R^{11}$ radicals independently of one another are H, $C_1$-$C_4$-alkyl, $C_1$-$C_4$-alkoxy or halogen,

$R^{12}$ is hydrogen, $C_1$-$C_4$-alkyl, $CHF_2$ or $CH_2CF_3$,

$R^{13}$ and $R^{14}$ independently of one another are H, $C_1$-$C_4$-alkyl, $C_2$-$C_4$-alkenyl or $C_3$-$C_4$-alkynyl,

$R^{15}$ is hydrogen or $C_1$-$C_4$-alkyl,

$R^{16}$ is hydrogen or $C_1$-$C_4$-alkyl,

E is CH or N,

G is $CH_2$ or O, and

Z is O or S, or a salt thereof.

**2.** A compound of the formula (I) or a salt thereof as claimed in claim 1, wherein A is a direct bond or a divalent radical of the formula $CH_2CH_2$, $CRR'$, $CH_2CHR$ or $CH_2CRR'$, where R and R' independently of

one another are $C_1$-$C_4$-alkyl or $C_2$-$C_4$-alkenyl.

**3.** A compound of the formula (I) or a salt thereof as claimed in claim 1 or 2, wherein $R^1$ is $C_1$-$C_4$-alkyl, a $C_1$-$C_4$-alkyl radical which is monosubstituted or polysubstituted by halogen or monosubstituted or disubstituted by $C_1$-$C_4$-alkoxy, $C_2$-$C_3$-alkenyloxy, $C_2$-$C_3$-alkynyloxy, $C_1$-$C_4$-alkylthio, $C_1$-$C_4$-alkylsulfinyl, $C_1$-$C_4$-alkylsulfonyl, phenyl or a phenyl radical which is monosubstituted to trisubstituted by radicals from the group comprising halogen, $C_1$-$C_4$-alkyl, $C_1$-$C_4$-alkoxy, $CF_3$, ($C_1$-$C_4$-alkoxy)carbonyl and nitro, or $R^1$ is $C_3$-$C_8$-cycloalkyl or a $C_3$-$C_8$-cycloalkyl radical which is monosubstituted or polysubstituted by halogen or monosubstituted to disubstituted by $C_1$-$C_4$-alkoxy or $C_1$-$C_4$-alkylthio, or $R^1$ is $C_5$-$C_8$-cycloalkenyl, cyclopropylmethyl, epoxypropyl, furfuryl, tetrahydrofurfuryl, benzyl, phenyl, or is a benzyl or phenyl radical which is substituted in the phenyl ring by one or more radicals from the group comprising halogen, $C_1$-$C_4$-alkyl, $C_1$-$C_4$-alkoxy, $CF_3$, ($C_1$-$C_4$-alkoxy)-carbonyl and nitro, or $R^1$ is a radical of the formula $NR^5R^6$, where $R^5$ is $C_1$-$C_4$-alkyl and $R^6$ is hydrogen or $C_1$-$C_4$-alkyl, or $R^5$ and $R^6$ together form an alkylene chain $-(CH_2)_n-$ with n being 4 or 5.

**4.** A compound of the formula (I) or a salt thereof as claimed in one or more of claims 1 to 3, wherein $R^2$ is $C_1$-$C_4$-alkyloxy, a $C_1$-$C_4$-alkyloxy radical which is monosubstituted or polysubstituted by halogen or monosubstituted or disubstituted by $C_1$-$C_4$-alkoxy, $C_2$-$C_4$-alkenyloxy, propargyloxy, $C_1$-$C_4$-alkylthio, $C_1$-$C_4$-alkylsulfinyl, $C_1$-$C_4$-alkylsulfonyl, ($C_1$-$C_4$-alkoxy)carbonyl, phenoxycarbonyl, benzyloxycarbonyl or phenyl, or is $C_3$-$C_8$-cycloalkyloxy.

**5.** A compound of the formula (I) or a salt thereof as claimed in one or more of claims 1 to 4, wherein $R^4$ is a radical of the formula

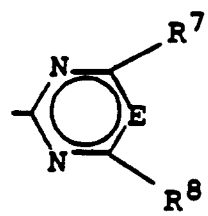

and

| | |
|---|---|
| $R^7$ and $R^8$ | independently of one another are halogen, $C_1$-$C_4$-alkyl, $C_1$-$C_4$-alkoxy, $C_1$-$C_4$-alkylthio or one of the last three abovementioned radicals which are monosubstituted or polysubstituted by halogen or monosubstituted or disubstituted by $C_1$-$C_4$-alkoxy or $C_1$-$C_4$-alkylthio, or $R^7$ and $R^8$ are a radical $NR^{13}R^{14}$, $C_3$-$C_6$-cycloalkyl, $-OCHR^{15}COOR^{16}$, allyl, propargyl, allyloxy or propargyloxy, |
| $R^{13}$ and $R^{14}$ | independently of one another are H or $C_1$-$C_4$-alkyl, |
| $R^{15}$ | is H or $C_1$-$C_4$-alkyl, |
| $R^{16}$ | is $C_1$-$C_4$-alkyl, and |
| E | is CH or N. |

**6.** A compound of the formula (I) or a salt thereof as claimed in one or more of claims 1 to 5, wherein

| | |
|---|---|
| A | is a direct bond, |
| $R^1$ | is $C_1$-$C_4$-alkyl, a $C_1$-$C_4$-alkyl radical which is monosubstituted or polysubstituted by halogen or monosubstituted to disubstituted by $C_1$-$C_4$-alkoxy, or is a radical of the formula $NR^5R^6$ where $R^5$ is $C_1$-$C_4$-alkyl and $R^6$ is hydrogen or $C_1$-$C_4$-alkyl or $R^5$ and $R^6$ together form an alkylene chain $-(CH_2)_n-$ with n being 4 or 5, |
| $R^2$ | is $C_1$-$C_4$-alkoxy which is unsubstituted or monosubstituted or polysubstituted by halogen or by ($C_1$-$C_4$-alkoxy)carbonyl, phenoxycarbonyl, benzyloxycarbonyl or phenyl, |
| $R^3$ | is H, $C_1$-$C_4$-alkyl or allyl, |
| $R^4$ | is a radical of the formula |

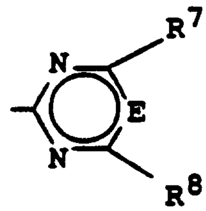

| | |
|---|---|
| $R^7$ and $R^8$ | independently of one another are halogen, $C_1$-$C_4$-alkyl, $C_1$-$C_4$-alkoxy or one of the last two abovementioned radicals which is halogenated, |
| E | is CH or N, and |
| Z | is an oxygen atom. |

**7.** A compound of the formula (I) or a salt thereof as claimed in one or more of claims 1 to 5, wherein

| | |
|---|---|
| A | is a radical of the formula $-CH_2-CH_2-$, |
| $R^1$ | is $C_1$-$C_4$-alkyl, a $C_1$-$C_4$-alkyl radical which is monosubstituted or polysubstituted by halogen or monosubstituted to disubstituted by $C_1$-$C_4$-alkoxy, or is $C_3$-$C_8$-cycloalkyl which is unsubstituted or monosubstituted or polysubstituted by halogen, or is benzyl, phenyl or a benzyl or phenyl radical which is monosubstituted or polysubstituted in the phenyl ring by halogen, $C_1$-$C_4$-alkyl, $C_1$-$C_4$-alkoxy, $CF_3$, ($C_1$-$C_4$-alkoxy)-carbonyl or nitro, |
| $R^2$ | is $C_1$-$C_4$-alkoxy which is unsubstituted or monosubstituted or polysubstituted by halogen or by ($C_1$-$C_4$-alkoxy)carbonyl, phenyloxycarbonyl, benzyloxycarbonyl or phenyl, |
| $R^3$ | is H, $C_1$-$C_4$-alkyl or allyl, |
| $R^4$ | is a radical of the formula |

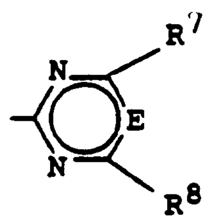

| | |
|---|---|
| $R^7$ and $R^8$ | independently of one another are halogen, $C_1$-$C_4$-alkyl, $C_1$-$C_4$-alkoxy or one of the last two abovementioned radicals which is halogenated, |
| E | is CH or N, and |
| Z | is O or S. |

**8.** A process for the preparation of a compound of the general formula (I) or a salt thereof as claimed in claim 1, which comprises

(a) reacting a compound of the formula (II)

$R^1$-Y-A-$NR^2$-$SO_2$-N = C = Z (II)

with a compound of the formula (III)

H-$NR^3R^4$ (III)

where, in formulae (II) and (III), A, Y, Z, $R^1$, $R^2$, $R^3$ and $R^4$ are as defined for formula (I), or

(b) reacting a compound of the formula (IV)

$R^1$-Y-A-$NR^2$-$SO_2$-$NH_2$ (IV)

with a carbamate, or thiocarbamate, of the formula (V)

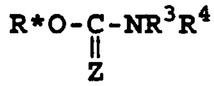 (V)

where, in formulae (IV) and (V), $R^1$, $R^2$, $R^3$, $R^4$, A, Y and Z are as defined for formula (I) and R* is $C_1$-$C_6$-alkyl, $C_1$-$C_4$-haloalkyl, phenyl or a phenyl radical which is monosubstituted or polysubstituted by halogen, $C_1$-$C_4$-alkyl or nitro, or
(c) reacting a carbamate, or thiocarbamate, of the formula (VI)

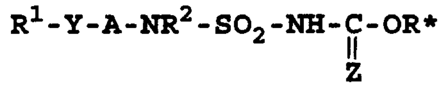 (VI)

with a compound of the formula (III) mentioned under (a), where $R^1$, $R^2$, R*, Y, A and Z are as defined, or
(d) reacting a compound of the formula (VII) or (VIII)

$R^1$-Y-A-NH-$R^2$ (VII)

$R^1$-Y-A-NH-$R^2$ x HCl (VIII)

with a compound of the formula (IX)

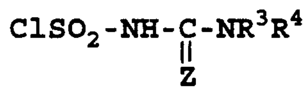 (IX)

where, in formulae (VII) to (IX), A, Y, Z, $R^1$, $R^2$, $R^3$ and $R^4$ are as defined above.

**9.** A herbicidal or plant-growth-regulating agent which contains a compound of the formula (I) or a salt thereof as claimed in one or more of claims 1 to 6 or 1 to 5 and 7, besides inert carrier substances.

**10.** The use of a compound of the formula (I) or a salt thereof as claimed in one or more of claims 1 to 6 or 1 to 5 and 7 as herbicides or plant growth regulators.

**11.** A compound of the formula (II)

$R^1$ - Y - A - $NR^2$ - $SO_2$ - N = C = Z (II)

where
$R^1$, $R^2$, A and Z are as defined for formula (I) in claim 1.

**12.** A process for the preparation of a compound of the formula (II) as claimed in claim 11, which comprises reacting a compound of the formula (VII)

$R^1$ - Y - A - NH - $R^2$ (VII)

where
$R^1$-Y-A and $R^2$ are as defined in claim 11, with chlorosulfonyl isocyanate.

**13.** A compound of the formula (VI)

$$R^1\text{-}Y\text{-}A\text{-}NR^2\text{-}SO_2\text{-}NH\text{-}\overset{\overset{\displaystyle Z}{\|}}{C}\text{-}OR^* \qquad (VI)$$

where
$R^1$, Y, A, $R^2$ and Z are as defined for formula (I) in claim 1 and
$R^*$ is $C_1$-$C_6$-alkyl, $C_1$-$C_4$-haloalkyl, phenyl or a phenyl radical which is monosubstituted or polysubstituted by halogen, $C_1$-$C_4$-alkyl or nitro.

**14.** A process for the preparation of the compound of the formula (VI) as claimed in claim 13, which comprises reacting a compound of the formula (IV)

$R^1$-Y-A-$NR^2$-$SO_2$-NH (IV)

with chloroformic esters of the formula

$$Cl\text{-}\overset{\overset{\displaystyle Z}{\|}}{C}\text{-}OR^*$$

where
$R^1$, A, Y, $R^2$ and $R^*$ in the above formulae are as defined in claim 13.

**Revendications**

**1.** Composés de formule générale I ci-dessous

$$R^1\text{-}Y\text{-}A\text{-}NR^2\text{-}SO_2\text{-}NH\text{-}\overset{\overset{\displaystyle Z}{\|}}{C}\text{-}NR^3R^4 \qquad (I)$$

formule dans laquelle

- A désigne une liaison directe ou un radical hydrocarboné en $C_1$-$C_{10}$ saturé ou insaturé, linéaire ou ramifié,
- $R^1$ un $C_1$-$C_8$-alkyle, un $C_2$-$C_8$-alcényle, un $C_2$-$C_8$-alcynyle, un $C_3$-$C_8$-cycloalkyle, un $C_5$-$C_8$-cycloalcényle ou l'un de ces cinq radicaux avec un ou plusieurs substituants pris parmi des halogènes et des $C_1$-$C_6$-alcoxy, $C_2$-$C_6$-alcényloxy, $C_2$-$C_6$-alcynyloxy, $C_1$-$C_6$-alkyl-thio, $C_1$-$C_6$-alkylsulfinyles, $C_1$-$C_6$-alkylsulfonyles et $C_3$-$C_6$-cycloalkyles, le radical d'un hétérocycle saturé trigonal à hexagonal avec un atome d'oxygène du cycle, un furyle, un phényle et un phényle avec un ou plusieurs substituants pris parmi des halogènes et des groupes CN, $C_1$-$C_4$-alkyles, $C_1$-$C_4$-alcoxy, $C_1$-$C_4$-halogénoalkyles tels que $CF_3$, ($C_1$-$C_3$-alcoxy)-carbonyles et nitro, ou bien un phényle ou un phényle avec un ou plusieurs substituants pris parmi les halogènes et des groupes CN, $C_1$-$C_4$-alkyles, $C_1$-$C_4$-alcoxy, $C_1$-$C_4$-halogénoalkyles tels que $CF_3$, ($C_1$-$C_3$-alcoxy)-carbonyles et nitro, ou encore un radical $-NR^5R^6$, $R^5$ et $R^6$ étant indépendamment l'un de l'autre l'hydrogène ou un $C_1$-$C_8$-alkyle ou bien l'un d'eux est un $C_1$-$C_4$-alcoxy, ou $R^5$ et $R^6$ forment ensemble une chaîne alkylène $(CH_2)n$-, n étant un nombre de 2 à 7;
- Y représente S, SO ou $SO_2$ ;
- $R^2$ un $C_1$-$C_8$-alkyloxy, un $C_2$-$C_8$-alcényloxy, un $C_2$-$C_8$-alcynyloxy ou l'un de ces trois radicaux avec un ou plusieurs substituants pris parmi les halogènes et des $C_1$-$C_6$-alcoxy, $C_2$-$C_6$-alcényloxy, $C_2$-$C_6$-alcynyloxy, $C_1$-$C_6$-alkylthio, $C_1$-$C_6$-alkylsulfinyles, $C_1$-$C_6$-alkylsulfonyles, -($C_1$-$C_3$-alcoxy)-carbonyles, phénoxycarbonyles, benzyloxycarbonyles et phényle, un $C_3$-$C_8$-

cycloalkyloxy sans substituants ou avec un ou plusieurs atomes d'halogènes ou un ou deux C1-C4-alcoxy ou C1-C4-alkylthio, un C5-C8-cycloalcényloxy,un cyclopropylmèthyloxy, époxy-propyloxy, furfuryloxy, tétrahydrofurfuryloxy, phenoxy-C1-C6-alkyloxy, phénoxy ou l'un de ces deux derniers radicaux avec sur le cycle phenylique un halogène, un $C_1$-$C_4$-alkyle, un $C_1$-$C_4$-alcoxy ou un groupe nitro ;

$R^3$ représente H, un $C_1$-$C_8$-alkyle, un $C_2$-$C_8$-alcényle, un $C_2$-$C_8$-alcynyle ou un C1-C4-alcoxy,

$R^4$ un radical de formule

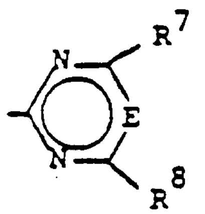
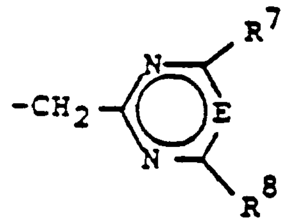
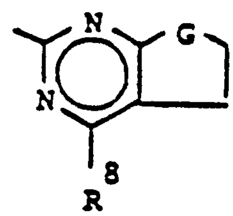
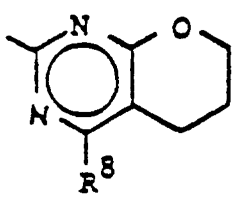
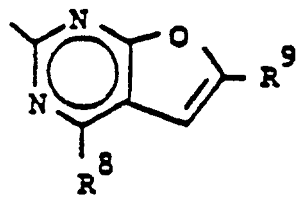
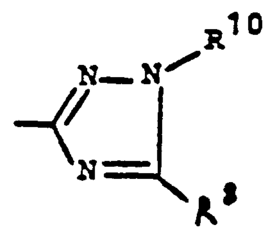
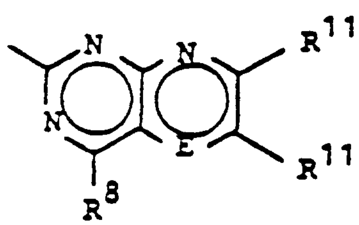

ou

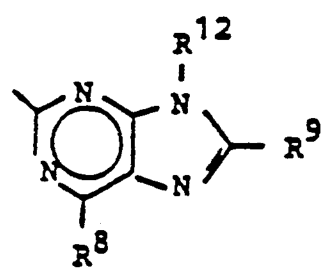

formules dans lesquelles

$R^7$ et R8 indépendamment l'un de l'autre représentent H, un halogène, un $C_1$-$C_6$-alkyle, un $C_1$-$C_6$-alcoxy, un $C_1$-$C_6$-alkylthio ou l'un de ces trois derniers radicaux avec comme substituants un ou plusieurs atomes d'halogène ou un ou deux $C_1$-$C_4$-alcoxy ou $C_1$-$C_4$-alkylthio, ou bien un radical $NR^{13}R^{14}$, $C_3$-$C_6$-cycloalkyle, $-OCHR^{15}COOR^{16}$, $C_3$-$C_5$-alcényle, $C_2$-$C_4$-alcynyle, $C_3$-$C_5$-alcényloxy ou $C_3$-$C_5$-alcynyloxy,

$R^9$ représente l'hydrogène ou un $C_1$-$C_4$-alkyle,

$R^{10}$ un $C_1$-$C_4$-alkyle, $-CHF_2$ ou $-CH_2F_3$,

les $R^{11}$ représentent indépendamment H, un $C_1$-$C_4$-alkyle, un $C_1$-$C_4$-alcoxy ou un halogène,

$R^{12}$ représente l'hydrogène ou un $C_1$-$C_4$-alkyle, $CHF_2$ ou $CH_2CF_3$,

$R^{13}$ et $R^{14}$ représentent indépendamment H, un $C_1$-$C_4$-alkyle, un $C_2$-$C_4$-alcényle ou un $C_3$-$C_4$-alcynyle,

$R^{15}$ représente l'hydrogène ou un $C_1$-$C_4$-alkyle,

$R^{16}$ représente l'hydrogène ou un $C_1$-$C_4$-alkyle,

E représente CH ou N et

G $CH_2$ ou O, et

Z représente un atome d'oxygène ou de soufre.

**2.** Composés de formule I et leurs sels selon la revendication 1, caractérisés en ce que A est une liaison directe ou un radical divalent $CH_2CH_2$, CRR', $CH_2CHR$ ou $CH_2CRR'$, R et R' étant, indépendamment l'un de l'autre, un alkyle en $C_1$-$C_4$ ou un alcényle en $C_2$-$C_4$.

**3.** Composés de formule I et leurs sels selon la revendication 1 ou 2, caractérisés en ce que $R^1$ est un $C_1$-$C_4$-alkyle avec éventuellement comme substituants un ou plusieurs atomes d'halogènes ou un ou deux $C_1$-$C_4$-alcoxy, $C_2$-$C_3$-alcényloxy, $C_2$-$C_3$-alcynyloxy, $C_1$-$C_4$-alkylthio, $C_1$-$C_4$-alkylsulfinyles, $C_1$-$C_4$-alkyl-sulfonyles, phényles ou phényles avec de un à trois substituants pris parmi les halogènes et des groupes $C_1$-$C_4$-alkyles, $C_1$-$C_4$-alcoxy, $CF_3$, ($C_1$-$C_4$-alcoxy)-carbonyles et nitro, ou bien un $C_3$-$C_8$-cycloalkyle avec éventuellement comme substituants un ou plusieurs atomes d'halogènes ou un ou deux $C_1$-$C_4$-alcoxy ou $C_1$-$C_4$-alkylthio, ou encore un $C_1$-$C_8$-cycloalcényle, un cyclopropylméthyle, époxypropyle, furfuryle, tétrahydrofurfuryle, benzyle, phényle ou benzyle ou phényle avec sur le cycle phénylique un ou plusieurs substituants pris parmi les halogènes et des groupes $C_1$-$C_4$-alkyles, $C_1$-$C_4$-alcoxy, $CF_3$, ($C_1$-$C_4$-alcoxy)-carbonyles et nitro, ou un radical - NR5R6 dont R5 est un $C_1$-$C_4$-alkyle et $R^6$ l'hydrogène ou un $C_1$-$C_4$-alkyle, ou bien $R^5$ et $R^6$ forment ensemble une chaîne alkylène $-(CH_2)_n-$, n étant le nombre 4 ou 5.

**4.** Composés de formule I et leurs sels selon une ou plusieurs des revendications 1 à 3, caractérisés en ce que $R^2$ est un $C_1$-$C_4$-alcoxy avec éventuellement comme substituants un ou plusieurs atomes d'halogènes ou un ou deux $C_1$-$C_4$-alcoxy, $C_2$-$C_4$-alcényloxy, propargyloxy, $C_1$-$C_4$-alkylthio, $C_1$-$C_4$-alkylsulfinyles, $C_1$-$C_4$-alkyl-sulfonyles, ($C_1$-$C_4$-alcoxy)-carbonyles, phénoxy-carbonyles, benzyloxy-carbonyles ou phényles, ou bien un $C_3$-$C_8$-cycloalkyloxy.

**5.** Composés de formule I et leurs sels selon une ou plusieurs des revendications 1 à 4, caractérisés en ce que

$R^4$ est un radical

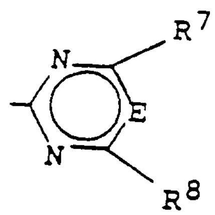

dans lequel

$R^7$ et R8 sont chacun indépendamment l'un de l'autre un halogène, un $C_1$-$C_4$-alkyle, un $C_1$-$C_4$-alcoxy, un $C_1$-$C_4$-alkylthio ou l'un de ces trois derniers radicaux avec comme substituants un ou plusieurs atomes d'halogènes ou un ou deux $C_1$-$C_4$-alcoxy ou $C_1$-$C_4$-alkylthio, ou encore un radical $NR^{13}R^{14}$, $C_3$-$C_6$-cycloalkyle, $-OCHR^{15}COOR^{16}$, allyle, propargyle, allyloxy ou propargyloxy,

$R^{13}$ et $R^{14}$ étant chacun indépendamment H ou un $C_1$-$C_4$-alkyle,

$R^{15}$ H ou un $C_1$-$C_4$-alkyle,

$R^{16}$ un $C_1$-$C_4$-alkyle, et

E CH ou N.

**6.** Composés de formule I et leurs sels selon une ou plusieurs des revendications 1 à 5, caractérisés en ce

A est une liaison directe,

$R^1$ un $C_1$-$C_4$-alkyle pouvant éventuellement avoir comme substituants un ou plusieurs atomes d'halogènes ou un ou deux $C_1$-$C_4$-alcoxy, ou bien un radical $NR^5R^6$, $R^5$ est un $C_1$-$C_4$-alkyle et $R^6$ l'hydrogène ou un $C_1$-$C_4$-alkyle ou $R^5$ et $R^6$ forment ensemble une chaîne alkylène $-(CH_2)_n-$, n étant le nombre 4 ou 5,

$R^2$ est un $C_1$-$C_4$-alcoxy sans substituants ou avec un ou plusieurs halogènes ou groupes ($C_1$-$C_4$-alcoxy)-carbonyles, phényloxycarbonyles, benzyloxycarbonyles ou phényles,

$R^3$ l'hydrogène, un $C_1$-$C_4$-alkyle ou un allyle,

$R^4$ un radical

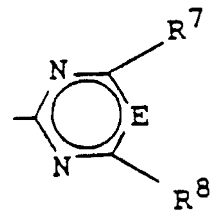

dans lequel

- $R^7$ et $R^8$ sont indépendamment l'un de l'autre un halogène, un alkyle ou un alcoxy $C_1$-$C_4$ ou l'un de ces deux radicaux halogénés, et
- E est CH ou N, et
- Z un atome d'oxygène.

**7.** Composés de formule I et leurs sels selon une ou plusieurs des revendications 1 à 5, caractérisés en ce que

- A est un radical $-CH_2-CH_2-$,
- $R^1$ un $C_1$-$C_4$-alkyle avec éventuellement comme substituants un ou plusieurs halogènes ou un ou deux $C_1$-$C_4$-alcoxy, un $C_3$-$C_8$-cycloalkyle non substitué ou avec un ou plusieurs halogènes, ou bien un benzyle ou un phényle avec éventuellement sur le cycle phénylique un ou plusieurs halogènes ou groupes $C_1$-$C_4$-alkyles, $C_1$-$C_4$-alcoxy, $CF_3$, ($C_1$-$C_4$-alcoxy)-carbonyles ou nitro,
- $R^2$ un $C_1$-$C_4$-alcoxy non substitué ou avec un ou plusieurs halogènes ou groupes ($C_1$-$C_4$-alcoxy)-carbonyles, phényloxycarbonyles, benzyloxycarbonyles ou phényles,
- $R^3$ est l'hydrogène, un $C_1$-$C_4$-alkyle ou un allyle,
- $R^4$ un radical de formule

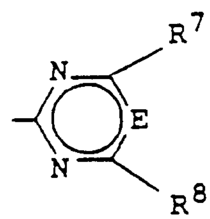

- $R^7$ et R8 étant chacun indépendamment un halogène, un alkyle ou un alcoxy en $C_1$-$C_4$, ou l'un de ces deux radicaux halogéné et
- E CH ou N, et
- Z représente l'oxygène ou le soufre.

**8.** Procédé de préparation des composés de formule I et de leurs sels selon la revendication 1, procédé caractérisé en ce que l'on fait réagir

(a) un composé de formule II

$R^1$-Y-A-$NR^2$-$SO_2$-N = C = Z (II),

avec un composé de formule III

H-$NR^3R^4$ (III),

les divers symboles ayant les mêmes significations que dans la formule I, ou bien on fait réagir

(b) un composé de formule IV

$R^1$-Y-A-$NR^2$-$SO_2$-$NH_2$ (IV)

avec un carbamate ou un thiocarbamate de formule V

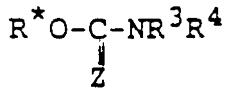 (V)

R*, dans la formule V, désignant un $C_1$-$C_6$-alkyle, un $C_1$-$C_4$-halogénoalkyle ou un phényle avec éventuellement comme substituants un ou plusieurs halogènes, $C_1$-$C_4$-alkyles ou groupes nitro, ou encore

(c) on fait réagir un carbamate ou un thiocarbamate de formule VI

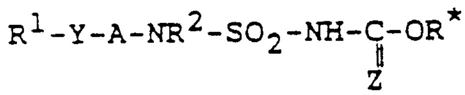 (VI)

avec un composé de formule III ci-dessus, les divers symboles ayant les significations indiquées, ou

(d) un composé de formule VII ou VIII

$R^1$-Y-A-NH-$R^2$ (VII)

$R^1$-Y-A-NH-$R^2$ x HCl (VIII)

avec un composé de formule IX

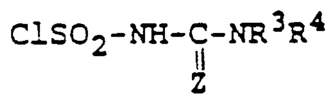 (IX)

les divers symboles des formules VII à IX ayant également les significations précédemment indiquées.

**9.** Produits herbicides ou régulateurs de la croissance de végétaux caractérisés en ce qu'ils comprennent un composé de formule I ou un sel de ce composé, selon une ou plusieurs des revendications 1 à 6 ou 1 à 5 et 7, avec des matières inertes servant de support.

**10.** L'emploi de composés de formule I ou de sels de ces composés selon une ou plusieurs des revendications 1 à 6 ou 1 à 5 et 7 comme produits herbicides ou régulateurs de la croissance de végétaux.

**11.** Les composés de formule II

$R^1$ - Y - A - $NR^2$ - $SO_2$ - N = C = Z (II)

dans laquelle $R^1$, $R^2$, A et Z ont les mêmes significations que dans la formule I.

**12.** Procédé de préparation des composés de formule II selon la revendication 11, procédé caractérisé en ce que l'on fait réagir un composé de formule VII

$R^1$ - Y - A - NH - $R^2$ (VII)

les divers symboles ayant les mêmes significations qu'à la revendication 11, avec l'isocyanate de chlorosulfonyle.

**13.** Les composés de formule VI

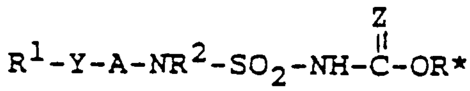

(VI)

dans laquelle
$R^1$, $R^2$, A, Y et Z ont les mêmes significations que dans la formule I et
$R^*$ désigne un $C_1$-$C_6$-alkyle, un $C_1$-$C_4$-halogénoalkyle, un phényle ou un phényle avec comme substituants un ou plusieurs halogènes ou groupes $C_1$-$C_4$-alkyles ou nitro.

**14.** Procédé de préparation des composés de formule VI selon la revendication 13, procédé caractérisé en ce que l'on fait réagir un composé de formule IV

$R^1$-Y-A-$NR^2$-$SO_2$-NH (IV)

avec un ester de l'acide chloroformique de formule

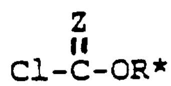

les divers symboles ayant les significations indiquées à la revendication 13.